# EUROPEAN PATENT SPECIFICATION

(11) **EP 0 295 051 B1**
(45) Date of publication and mention of the grant of the patent: **09.06.1993**
(21) Application number: 88305180.7
(22) Date of filing: 07.06.1988
(51) Int. Cl.: C07D 417/12, C07D 417/06, C07D 237/32, C07D 307/89

(54) **Preparation of oxophthalazinyl acetic acids having benzothiazole or other heterocyclic side chains**
Herstellung von Oxophthalazinylessigsäuren mit einer Benzothiazol- oder einer anderen heterocyclischen Seitenkette
Préparation d'acides oxophtalazinylacétiques ayant du benzothiazole ou d'autres groupes hétérocycliques comme chaînes latérales

(30) Priority: 09.06.1987 US 59899
(43) Date of publication of application: 14.12.1988
(73) Proprietor: PFIZER INC., New York, N.Y. 10017 (US)
(72) Inventor: Mylari, Banavara Lakshmana, Waterford Connecticut (US); Zembrowski, William James, Oakdale Connecticut (US)
(74) Representative: Wood, David John

(56) References cited:
- EP-A- 0 002 895
- EP-A- 0 222 576
- US-A- 4 298 530
- CHEMICAL ABSTRACTS, vol. 94, no. 9, 02.03.81, page 733, abstract 65 710s
- CHEMICAL ABSTRACTS, vol. 106, no. 9, 02.03.87, page 616, abstract 67 388s
- PATENT ABSTRACTS OF JAPAN, vol. 9, no. 160, 04.07.85, (C-289)(1883); & JP - A - 60 36 464
- CHEMICAL ABSTRACTS, vol. 102, no. 7, 18.02.85, page 595, abstracts 62 170p; A. SUGIMOTO et al.: "Synthesis and inhibitory effects on patelet aggregation of phthalazinone derivatives"
- CHEMICAL ABSTRACTS, vol. 78, no. 7, 19.02.73, page 130, abstract 39 222j; B.T. BROWN et al.: "New inhibitors of auxin transport"
- TETRAHEDRON LETTERS, vol. 25, no. 24, 1984, pages 2569-2572; N. TSUBONIWA et al.: "Reformatsky type reaction by means of Bu3SnA1Et2 or Bu3PbA1Et2"

## Description

The present invention relates to the preparation of oxophthalazinyl acetic acids having benzothiazole or other heterocyclic side chains. Such compounds function as aldose reductase inhibitors and are useful in the treatment of certain chronic complications arising from diabetes mellitus, such as diabetes cataracts, retinopathy and neuropathy. Such compounds are disclosed in European Patent Application Publication Number 0 222 576 of Pfizer Inc.

The present invention relates to a process for the preparation of a compound of the formula 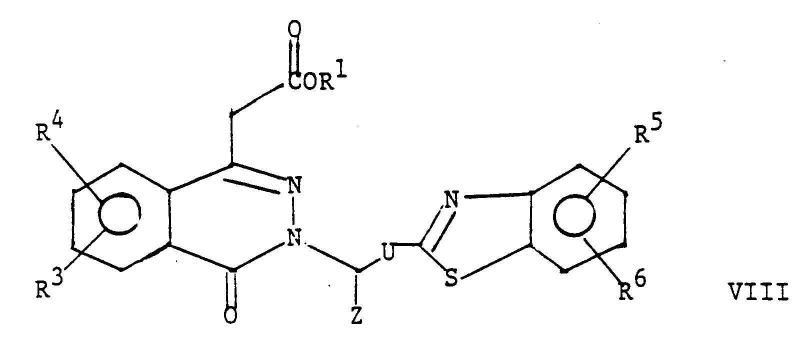
wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄-alkylthio, C₁-C₄ alkylsulfinyl, C₁-C₄ alkylsulfonyl, or nitro, or R³ and R⁴ taken together are C₁-C₄ alkanedioxy; R⁵ and R⁶ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, nitro, or benzo or R⁵ and R⁶ taken together are C₁-C₄ alkanedioxy; Z is hydrogen or methyl; and U is S, CH₂ or a covalent bond, comprising reacting a compound of the formula 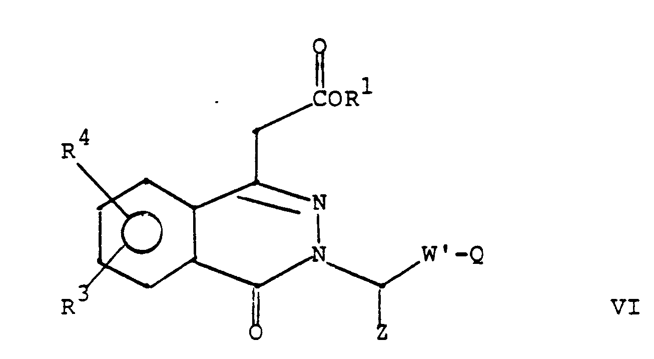
wherein R¹, R³, R⁴ and Z are as defined above, W′ is S, CH₂ or a covalent bond, and Q is -CN or 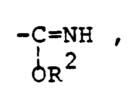
wherein R² is C₁-C₄ alkyl, or an acid addition salt thereof with a compound of the formula 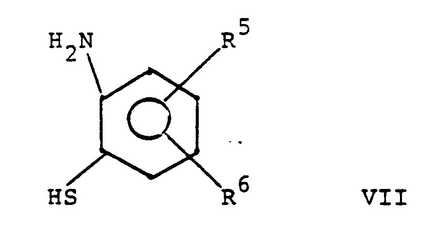
wherein R⁵ and R⁶ are defined above, or with an acid addition salt thereof, with the proviso that when neither one of said compounds of the formula VI and formula VII are in the form of its acid addition salt when it is added to the other of said compounds, the reaction is conducted in the presence of a strong acid; and, if desired, hydrolyzing a compound of the formula VIII wherein R¹ is other than hydrogen to the corresponding compound of the formula VIII wherein R¹ is hydrogen. The compounds of the formula VI wherein Q is 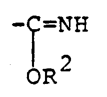
and all compounds of the formula VII may form acid addition salts. Although, we do not wish to be bound by theory, we believe that the addition of a strong acid to the aforementioned reaction mixture results in the formation in situ of an acid addition salt of at least one (and possibly both) of the compounds of the formula VI and formula VII and that the acid addition salt subsequently reacts with the other compound. Thus, at least one of the reactants is an acid addition salt that is preformed or formed in situ.

The present invention also relates to the foregoing compounds of the formula VI, to processes for preparing intermediates useful in the preparation of compounds of the formula VI, to intermediates useful in preparing compounds of the formula VI, and to other processes wherein such intermediates are useful.

The intermediates of the present invention include the compounds of the formula II described below and also include compounds of the formula 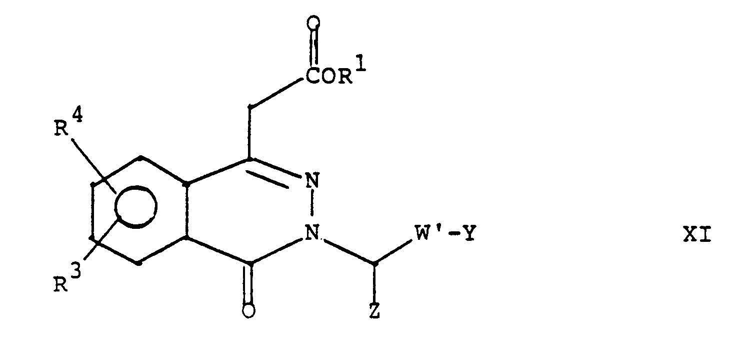
wherein R¹ is hydrogen or C₁ to C₄ alkyl; and R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; Z is hydrogen or methyl; W′ is S, CH₂ or a covalent bond; and Y is hydroxy, chloro, bromo, cyano or 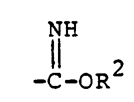
wherein R² is C₁-C₄ alkyl, with the proviso that when W′ is S, Y cannot be hydroxy, chloro or bromo.
More specifically, compounds of the formula XI include compounds wherein:
(a) R¹ is methyl or ethyl, R³ and R⁴ are hydrogen, W′ is a covalent bond or CH₂, and Y is CN; (b) R¹ is methyl or ethyl, R³ and R⁴ are hydrogen, Y is CN, W′ is a covalent bond and Z is hydrogen; (c) R¹ is ethyl, W′ is CH₂, Y is CN, and Z is hydrogen; (d) R¹ is methyl or ethyl, R³ and R⁴ are hydrogen, and Y is chloro or bromo, and the foregoing compounds wherein Z is hydrogen; (e) R¹ is methyl or ethyl, R³ and R⁴ are hydrogen, and Y is hydroxy, and the foregoing compounds wherein Z is hydrogen; (f) Y is 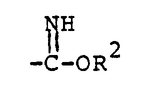
wherein R² is C₁-C₄ alkyl; and (g) R³ and R⁴ are hydrogen and Y is 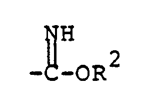
wherein R² is ethyl.

The aforementioned compound of the formula VI wherein Q is -CN may be prepared by reacting a compound of the formula 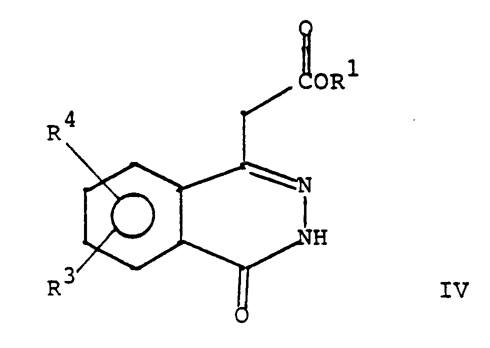
wherein R¹, R³ and R⁴ are as defined for formula VIII, with a compound of the formula 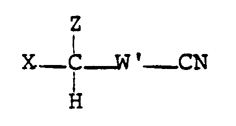
wherein X is chloro, bromo, -OSO₂-(C₁ to C₄ alkyl), or -OSO₂-aryl wherein aryl is phenyl, naphthyl, substituted phenyl, or substituted naphthyl, wherein the substituents on the substituted phenyl and substituted naphthyl groups are C₁ to C₄ alkyl, halogen or nitro; Z is hydrogen or methyl and W' is CH₂ or a covalent bond in the presence of a suitable base. The base should be of sufficient strength to catalyze the desired nucleophilic displacement. Examples of suitable bases include alkali metal hydrides (e.g., sodium hydride), alkali metal carbonates (e.g., potassium carbonate) alkali metal hydroxides (e.g., sodium hydroxide or potassium hydroxide), and alkali metal alkoxides (e.g., potassium tert-butoxide or sodium methoxide). A suitable solvent for this reaction is DMF (dimethylformamide). The reaction temperature is preferably about 20 to about 100°C, more preferably about 40°C.

Alternatively, the compound of the formula VI wherein W′ is a covalent bond, Z is hydrogen and Q is -CN may be prepared by reacting a compound of the formula 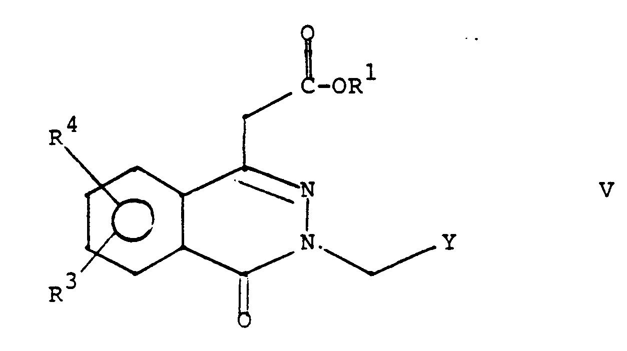
wherein Y is chloro or bromo; and R¹, R³ and R⁴ are as defined for formula VIII, with an alkali metal cyanide or alkaline earth metal cyanide. Examples of suitable cyanides include sodium and potassium cyanide. Suitable solvents include C₁-C₄ alcohols, C₁-C₄ alkanones and polar aprotic solvents e.g., DMF. The reaction temperature is preferably about 0 to about 40°C, more preferably about 20°C.

Compounds of the formula VI wherein W' is sulfur, Z is hydrogen and Q is -CN may be prepared by reacting a compound of the formula V wherein Y is chloro or bromo; and R¹, R³ and R⁴ are as defined for formula VIII, with an alkali metal or ammonium thiocyanate. Suitable solvents include C₁-C₄ alcohols, C₁-C₄ alkanones and polar aprotic solvents, e.g., DMF. The reaction temperature is preferably about 0 to about 40°C, more preferably about 20°C.

Compounds of the formula V where Y is chloro or bromo are, in turn, prepared from compounds of the formula V wherein Y is hydroxy by reaction with phosphorous trichloride or phosphorous tribromide at a temperature of about 0 to about 40°C, preferably about 20°C.

The aforementioned compound of the formula IV may be prepared by reacting a compound of the formula 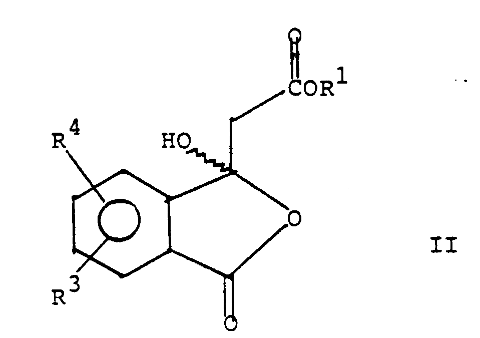
wherein R¹ is as defined above, with NH₂NH₂.

Alternatively, the compound of the formula II may be reacted with a strong acid (e.g., sulfuric acid or p-toluene sulfonic acid) to form a compound of the formula 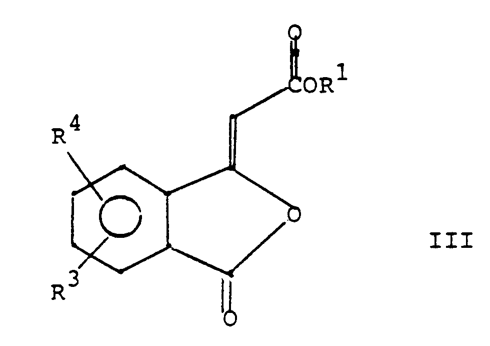
wherein R¹ is as defined for formula VIII, and the compound of formula III may be reacted with NH₂NH₂ to form a compound of formula IV.

Compounds of the formula V wherein Z is H, W is a covalent bond and Y is chloro or bromo are also useful in preparing compounds of the formula 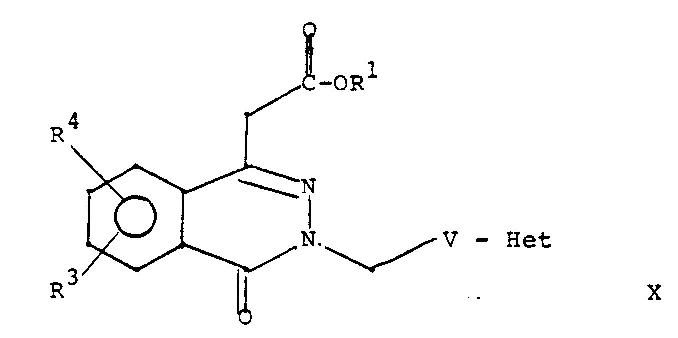
wherein R¹, R³ and R⁴ are as defined above for formula VIII; V is oxygen, sulfur or NH; and Het is a heterocyclic 5-membered ring having one nitrogen, oxygen or sulfur, two nitrogens one of which may be replaced by oxygen or sulfur, or three nitrogens one of which may be replaced by oxygen or sulfur, said ring substituted by one or two fluoro, chloro, C₁-C₄ alkyl or phenyl or condensed with benzo, or substituted by one of pyridyl, furyl or thienyl, said phenyl or benzo optionally substituted by one of iodo or trifluoromethylthio, or one or two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro, chloro, bromo, C₁-C₄ alkyl or C₁-C₄ alkoxy; a heterocyclic 6-membered ring having one to three nitrogen atoms, or one or two nitrogen atoms and one oxygen or sulfur, and said ring substituted by one or two C₁-C₄ alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro, chloro, C₁-C₄ alkyl or C₁-C₄ alkoxy; oxazole or thiazole condensed with a 6-membered aromatic group containing one or two nitrogen atoms or with thiophene or furane, each optionally substituted by one of fluoro, chloro, bromo, trifluoromethyl, methylthio or methylsulfinyl; imidazolopyridine; naphthothiazole; or naphthoxazole. Such compounds are disclosed in European Patent Application Publication Number 0 222 576 of Pfizer Inc. The foregoing compounds of the formula X are prepared by reacting a compound of the formula V wherein Z is hydrogen, W' is a covalent bond and Y is chloro or bromo with an appropriate heterocycle containing an OH, SH, or NH₂ group in an aqueous, alcoholic, or polar aprotic solvent. Examples of suitable solvents include ethanol and DMF. The reaction may be catalyzed by a suitable base. The base should be of sufficient strength to catalyze the desired nucleophilic displacement. Examples of suitable bases include alkali metal hydrides (e.g., sodium hydride), alkali metal carbonates (e.g., potassium carbonate), alkali metal hydroxides (e.g., sodium hydroxide or potassium hydroxide) and alkali metal alkoxides (e.g., potassium tert-butoxide or sodium methoxide). The reaction temperature is preferably about 30 to about 100°C, preferably about 40°C.

The processes of the present invention are illustrated by the following reaction scheme:

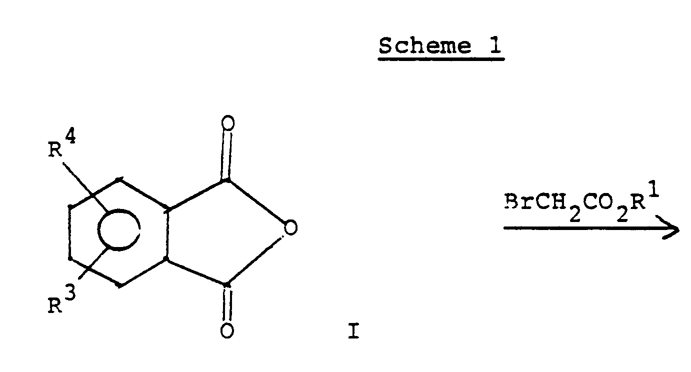

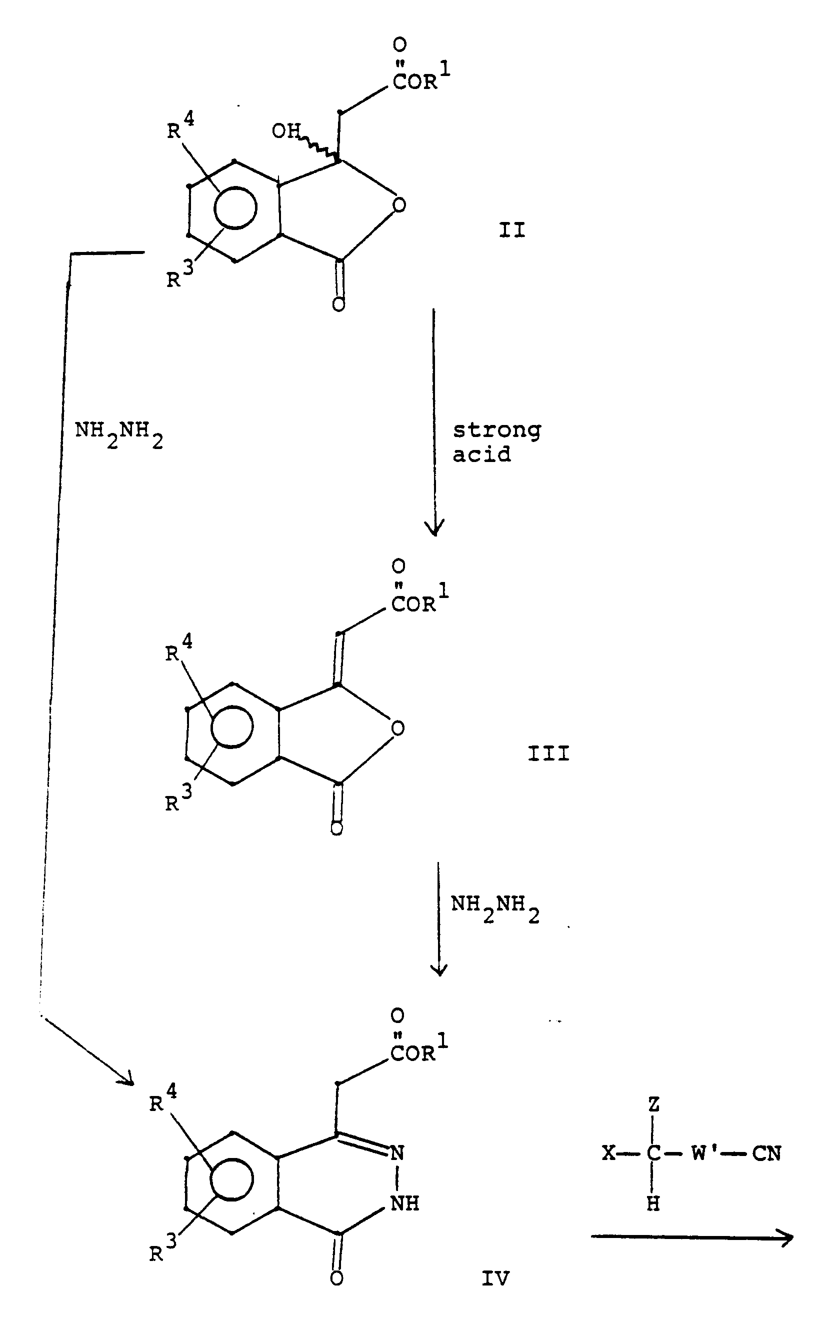

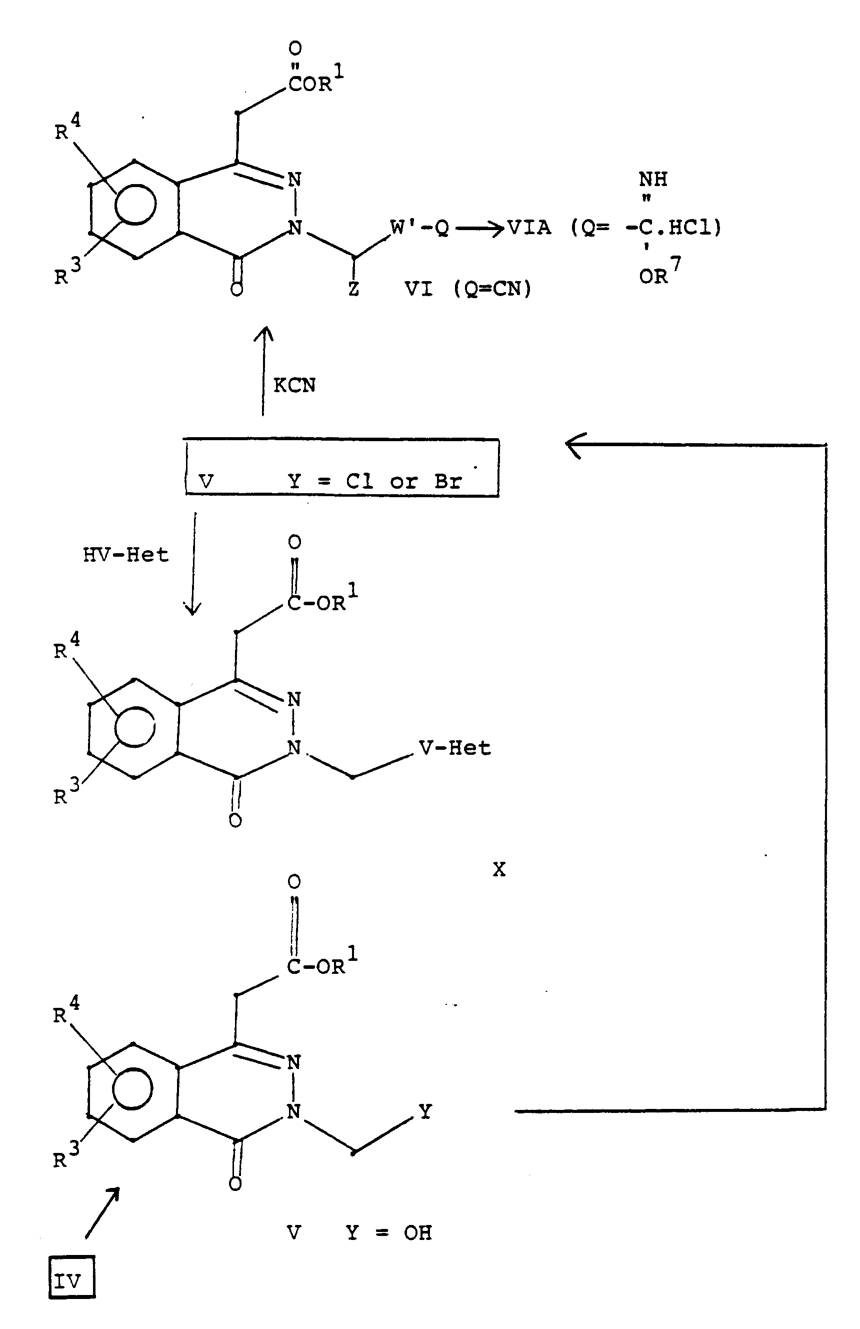

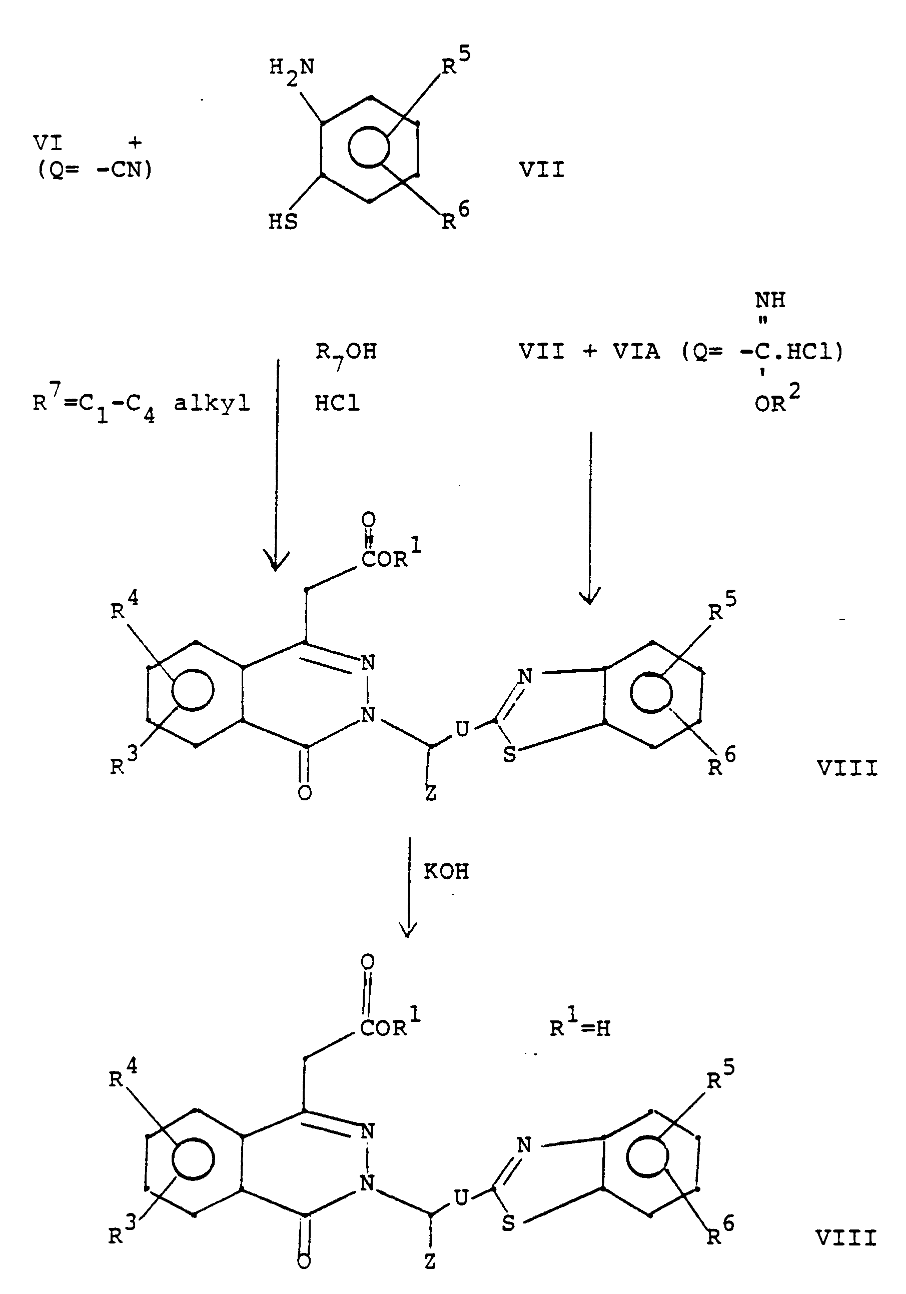

A compound of the formula I is converted to a compound of the formula II using standard Reformatsky reaction conditions with zinc or a zinc-copper couple or using a variety of well-known modifications of the Reformatsky reaction (see, for example Tetrahedron Letters, 2569 (1984)). Suitable solvents for the conversion of the compound of formula I to the compound of formula II include aromatic hydrocarbons (e.g., benzene) and dialkyl ethers and cyclic ethers (e.g., tetrahydrofuran). The temperature is preferably maintained at about 35 to about 100°C, more preferably, at reflux.

A compound of the formula II is converted to a compound of the formula III in the presence of a strong acid in a compatible solvent or without the use of a solvent. When a solvent is used, it is preferred that the solvent be a hydrocarbon solvent (e.g., benzene or toluene) and that the acid be an organic acid (e.g., p-toluenesulfonic acid). When such conditions are used, the temperature should preferably be at least about 90°C, more preferably, about 90 to about 100°C. The reaction mixture may conveniently be maintained at the reflux temperature of the reaction mixture.

When it is desired to convert a compound of the formula II to a compound of the formula III without using a solvent, it is preferred to use as the acid sulfuric acid at a temperature of about 0 to about 30°C, more preferably about 0 to about 20°C.

The conversion of a compound of the formula II or III to a compound of the formula IV may be accomplished with anhydrous or aqueous hydrazine in an alcoholic solvent (e.g., ethanol) at a temperature of about 20 to about 80°C, preferably about 60°C. Thus, the temperature may be room temperature or the reflux temperature of the solvent.

A compound of the formula IV may be converted to a compound of the formula V where Y is OH as described in Tetrahedron, 531 (1964). The reaction temperature is preferably about 20 to about 100°C, more preferably about 30 to 40°C.

In order to prepare a compound of the formula VIII wherein R¹ is C₁-C₄ alkyl, a compound of the formula VI wherein Q is -CN may be reacted with a preformed acid addition salt of a compound of the formula VII (e.g., a hydrochloride salt) or with an acid addition salt prepared in situ, for example, by the addition of a strong acid such as hydrochloric acid. In practice, it is convenient to use the preformed acid addition salt. The solvent is preferably a C₁ to C₄ alkanol (e.g., ethanol) but mixtures of at least one molar equivalent of C₁-C₄ alkanol with a hydrocarbon or halocarbon solvent may be used. Examples of hydrocarbon and halocarbon solvents include benzene and chloroform, respectively. The temperature is preferably at least about 60°C. The reaction mixture is conveniently maintained at reflux temperature.

When the foregoing reaction is carried out in C₁-C₄ alkanol solvents (e.g. ethanol), the resulting compound of the formula VIII wherein R¹ is C₁-C₄ alkyl can be hydrolyzed in situ directly to the compound of the formula VIII wherein R¹ is H. The hydrolysis may be accomplished by adding an aqueous solution of a base such as sodium hydroxide or potassium hydroxide. The temperature is preferably about 20 to about 100°C, preferably about 60°C.

If a compound of the formula VIII is prepared in a solvent other than a C₁-C₄ alkanol, the compound may be isolated, dissolved in a C₁-C₄ alkanol, and then hydrolyzed as described above.

The preparation of a compound of the formula VIII may also be carried out by co-melting a mixture of a compound of the formula VI with a preformed acid addition salt of a compound of the formula VII (e.g., a hydrochloride salt) at a temperature between about 110 and about 180°C.

In another method, compound of the formula VIII may be prepared by reacting a compound of the formula VI wherein Q is 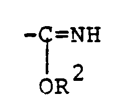
wherein R² is C₁-C₄ alkyl (hereinafter also referred to as a compound of the formula VIA) with a compound of the formula VII either as is or as in the form of an acid addition salt (e.g. hydrochloride salt). The reaction may be carried out in alcoholic, hydrocarbon or halocarbon solvents. Examples of such solvents include ethanol, toluene and chloroform, respectively. The reaction temperature is preferably at least about 60°C. The reaction mixture is conveniently maintained at reflux temperature. Alternatively the reaction may be carried out by co-melting a mixture of the compound of the formula VIA with a compound of the formula VII at a temperature between about 110 and about 180°C.

The compound of the formula VIA may be prepared by reacting a compound of the formula VI with a C₁-C₄ alkanol in the presence of a mineral acid or organic acid. A preferred example of a mineral acid is gaseous hydrogen chloride. An example of an organic acid is p-toluene sulfonic acid. The reaction may be carried out at a temperature of about -5°C up to about 40°C. If desired, the compound of the formula VIA may be prepared without adding an acid to the reaction mixture. In such a case, the reaction of the compound of the formula VIA and the compound of the formula VII should be conducted in the presence of a strong acid.

The reactions described above may be carried out at a pressure of about 0.5 to about 2 atmospheres (preferably at a pressure of about 1 atmosphere).

The compounds of formula VIII wherein R¹ is H and the pharmaceutically acceptable salts thereof are useful as inhibitors of the enzyme aldose reductase in the treatment of chronic complications of diabetes, such as diabetic cataracts, retinopathy and neuropathy. The compounds may be administered to a subject in need of treatment by a variety of conventional routes of administration, including orally, parenterally and topically. In general, these compounds will be administered orally or parenterally at dosages between about 0.5 and 25 mg/kg. body weight of the subject to be treated per day, preferably from about 1.0 to 10 mg/kg. However, some variation in dosage will necessarily occur depending on the condition of the subject being treated.

The following non-limiting Examples are illustrative of the present invention. All melting points are uncorrected.

### Example 1

### Ethyl-1,3-dihydro-1-hydroxy-3-oxo-1-isobenzofuran acetate

### Procedure A

To a refluxing slurry of zinc dust (37.0) in benzene (250 ml) was added a portion (15 ml) of a solution of ethyl bromoacetate (8.35 g) and phthalic anhydride (7.4 g) in benzene (250 ml). Following an exothermic reaction during this addition, the remaining portion of the benzene solution was added and the refluxing continued for 8 hours. The reaction mixture was cooled to room temperature and was then poured onto aqueous sulfuric acid (100 ml, 10%) and the organic layer was collected. This organic layer was sequentially washed with water (2 x 50 ml), aqueous sodium bicarbonate (25 ml, 10% by weight) and finally with water (25 ml). The washed organic extract was dried and evaporated. The resulting crude product was purified by chromatography (yield: 12.84 g; ¹HNMR(CDCl₃, 60MHz); 1.25(t, J-8Hz, 3H), 3.15 (s, 2H), 4.2 (9, J=8 Hz, 2H), 6.25 (broad, 1H), 7.6 (M, 4H)).

### Procedure B

To a refluxing suspension of zinc-copper couple (31.0 g) in tetrahydrofuran (50 ml) was gradually added a solution of phthalic anhydride (29.6 g) and ethyl bromoacetate (13.1 g) in tetrahydrofuran (150 ml). The reaction mixture was refluxed for 2 hours and it was then cooled and filtered. The filtrate was added to aqueous HCl (200 ml, 10% by volume) and then extracted with ethyl acetate (3 x 5 ml). The ethyl acetate layer was washed with water (2 x 50 ml) and evaporated to a clear colorless oil (yield: 18.2 g; ¹HNMR as in Procedure A).

### Example 2

### t-Butyl-1,3-dihydro-1-hydroxy-3-oxo-1-isobenzofuran acetate

Starting from phthalic anhydride (14.81 g), t-butyl bromoacetate (29.25 g), zinc-copper couple (15.0 G) and tetrahydrofuran (100 ml) and using the method of Procedure B, the title product was obtained (yield; 24.0 g). This product was further purified by chromatography (¹HNMR (CDCl, 250 MHz), 1.90 (s, 9H), 4.0 (s, 2H), 6.7 - 7.2 (m, 4H)).

### Example 3

### (Z)-3-Ethoxycarbonylmethylidenaphthalide,

Ethyl-1,3-dihydro-3-oxo-1-isobenzofuran acetate (2.36 g) was dissolved in concentrated sulfuric acid (5 ml) and allowed to stand at room temperature for 5 minutes. It was then slowly poured onto ice-water (50 ml) and the resulting white precipitate was collected, washed with water (3 x 25 ml) and then air-dried (yield: 2.08 g; See J. Org. Chem., 31, 4077, (1966).

### Example 4

### Ethyl-4-oxo-3H-phthalazin-1-yl-acetate

To a solution of ethyl-1,3-dihydro-3-oxo-1-isobenzofuran acetate (4.72 g) in ethanol (20 ml) was added hydrazine (1.28 g) all in one portion. The resulting white precipitate was collected, washed with aqueous HCl (20 ml, 10% by volume) and then with water. The solid was air-dried to obtain the title compound (See U.S. Patent 4,251,528).

### Example 5

### t-Butyl-4-oxo-3H-phthalazin-1-yl-acetate

To a solution of t-butyl-1,3-dihydro-1-hydroxy-3-oxo-1-isobenzofuran acetate (7.92 g) in ethanol (200 ml) was added hydrazine hydrate (5.0 ml, 85% by weight) and then refluxed for 1 hour. The precipitated white solid was collected, washed with water (2 x 25 ml) and then air-dried (yield: 5.30 g; m.p. 164-166°C).

### Example 6

### Methyl-3-cyanomethyl-4-oxo-3-H-phthalazin-1-ylacetate

### Procedure A

To a mixture of methyl-4-oxo-3-H-phthalazin-1-ylacetate (5.45 g) in dimethylformamide (25 ml) was added dry potassium t-butoxide (2.95 g) all in one portion. To the resulting dark green solution was gradually added chloroacetonitrile (1.89 g). The reaction mixture was stirred for 30 minutes and was then poured onto ice water (100 ml). Sufficient 10% HCl (by volume) was added to adjust the pH of the resulting mixture to about 4.0 and the precipitated off-white solid was collected and air-dried (yield: 5.74 g; m.p. 118-119°C).

### Procedure B

Procedure A was repeated as above except that chloroacetonitrile was replaced by bromoacetonitrile (3.0 g). The title product was obtained in 4.93 g yield with the same melting point as the product of Procedure A.

### Procedure C

Procedure A was repeated on twice the scale but chloroacetonitrile was replaced by cyanomethyl benzenesulfonate (10.90 g). The title product was obtained in 10.98 g yield.

### Example 7

### Ethyl-3-cyanomethyl-4-oxo-3-H-phthalazin-1-ylacetate

### Procedure A

To a solution of ethyl-4-oxo-3-H-phthalazin-1-ylacetate (11.31 g) and dry potassium t-butoxide (5.9 g) in dimethylformamide (50 ml) was added chloroacetonitrile (3.78 g) and the solution was stirred for 30 minutes. This solution was poured onto ice water (300 ml); sufficient 10% HCl was added to adjust the pH of the resulting mixture to about 4.0 and the precipitated solid was collected and air-dried (yield: 11.81 g; m.p. 113-114°C).

### Procedure B

Chloroacetonitrile (3.0 ml) was added to a solution of ethyl 4-oxo-3H-phthalazin-1-ylacetate (10.0 g) in dimethylformamide (100 ml) containing anhydrous potassium carbonate (9.0 g) and the mixture was stirred overnight. It was then poured onto ice-water (500 ml); sufficient 10% HCl was added to adjust the pH to about 4.0 and the precipitated solid was obtained upon air drying (yield: 9.7 g; m.p. 113-114°C).

### Procedure C

A mixture of ethyl-4-oxo-3H-phthalazin-1-ylacetate (5.0 g), potassium carbonate (4.5 g), acetone (100 ml) and chloroacetonitrile (2.0 ml) was refluxed overnight. The reaction mixture was then worked-up as in Procedure B (yield: 4.1 g).

### Procedure D

To an ice-cold solution of ethyl-3-bromomethyl-4-oxo-3-H-phthalazin-1-ylacetate (2.43 g) in acetone (3.5 ml) was added dropwise a solution of potassium cyanide (0.49 g) and potassium iodide (2 mg) in water (3.5 ml). The reaction mixture was stirred for 2 hours and it was then poured onto ice water (200 ml). The precipitated solid was purified by chromatography on silica gel, eluting with 95% CH₂Cl₂ - 5% ethyl acetate (percents by volume) to obtain the title compound (yield: 1.54 g).

### Example 8

### Ethyl-3-cyanoethyl-4-oxo-3-H-phthalazin-1-ylacetate

To a solution of ethyl-4-oxo-3-H-phthalazin-1-ylacetate (10.91 g) and dry potassium t-butoxide (5.90 g) in dimethylformamide (50 ml) was added 3-chloropropionitrile (4.92 g) and the resulting solution was stirred for 30 minutes. The solution was then poured onto ice-water (300 ml); sufficient 10% HCl was added to adjust the pH of the resulting mixture to about 4.0 and the precipitated solid was collected and crystallized from methanol (yield: 7.64 g; m.p. 125-126°C).

### Example 9

### Ethyl-3-hydroxymethyl-4-oxo-3-H-phthalazin-1-ylacetate

A mixture of ethyl-4-oxo-3-H-phthalazin-1-ylacetate (23.42 g), ethanol (200 ml) and aqueous formaldehyde (37% concentration, 100 ml) was refluxed for 40 hours. This solution was concentrated to 100 ml and was then poured onto ice water (750 ml). The precipitated solid was collected and air-dried (yield: 17.1 g; m.p. 113-114°C).

### Example 10

### Methyl-3-hydroxymethyl-4-oxo-3-H-phthalazin-1-ylacetate

A mixture of methyl-4-oxo-3-H-phthalazin-1-ylacetate (2.18 g), methanol (50 ml) and aqueous formaldehyde (37% concentration, 10 ml) was refluxed for 40 hours. The reaction mixture was cooled to room temperature and then poured onto water (50 ml). The resulting solid was collected and crystallized from methanol (yield: 0.5 g; m.p. 154-155°C).

### Example 11

### Ethyl-3-bromomethyl-4-oxo-3-H-phthalazin-1-ylacetate

A solution of ethyl-3-hydroxymethyl-4-oxo-3-H-phthalazin-1-ylacetate (13.1 g), phosphorous tribromide (13.5 g) and anhydrous ether (250 ml) was stirred overnight at room temperature. It was then poured onto water (200 ml). The organic layer was collected, washed again with water (3 x 100 ml) and then dried over anhydrous sodium sulfate. The dried organic extract was evaporated to dryness and the resulting crude solid was purified by chromatography on silica gel, eluting with 95% CH₂Cl₂ - 5% ethyl acetate (percents by volume) to obtain the title compound (yield: 9.8 g; m.p. 96°C).

### Example 12

### Methyl-3-bromomethyl-4-oxo-3-H-phthalazin-1-ylacetate

A solution of methyl-3-hydroxymethyl-4-oxophthalazin-1-ylacetate (0.49 g), phosphorous tribromide (0.54 g) and methylene chloride (10 ml) was stirred at room temperature for 2 hours. The solution was poured onto ice-water (5 ml) and the separated methylene chloride layer was collected, dried and evaporated to obtain a light yellow solid (yield: 0.43 g; m.p. 98-104°C).

### Example 13

### Ethyl-3-Chloromethyl-4-oxo-3-H-phthalazin-1-ylacetate

A solution of ethyl-3-hydroxymethyl-4-oxo-3-H-phthalazin-1-ylacetate (1.31 g) and methanesulfonyl chloride (0.69 g) in methylene chloride (10 ml) containing pyridine (0.8 ml) was stirred at room temperature overnight. Upon evaporation of methylene chloride and purification of the residue by chromatography on silica gel, eluting with a mixture of a chloroform and ethyl acetate (9:1), the title product was obtained (yield: 0.51 g; m.p. 99-100°C).

Alternatively, follow the procedure in Example 11, but substitute phosphorous trichloride for phosphorous tribromide, to obtain the title compound.

### Example 14

### Ethyl-3-(5-trifluoromethylbenzothiazol-2-ylmethyl)-4-oxo-3-H-phthalazin-1-ylacetate

### Procedure A

A mixture of ethyl 3-cyanomethyl-4-oxo-phthalazin-1-ylacetate (2.71 g), 2-amino-4-trifluoromethyl-thiophenol hydrochloride (2.40 g) and ethanol (20 ml) was refluxed for 8 hours. The heavy precipitate obtained upon cooling was filtered and the collected solid was air-dried to obtain the title compound (yield: 4.3 g; m.p. 136°C).

### Procedure B

A mixture of ethyl 3-cyanomethyl-4-oxo-phthalazin-1-ylacetate (0.27 g) and 2-amino-4-trifluoromethylthiophenol hydrochloride (0.23 g) was heated to a melt at 180°C for 10 minutes. The mobile liquid was cooled and then suspended in water. Filtration of the mixture gave the title compound in 75% yield.

### Example 15

### Ethyl-3-(5,7-difluorobenzothiazol-2-ylmethyl)-4-oxo-3-H-phthalazin-1-ylacetate

A mixture of ethyl-3-cyanomethyl-4-oxo-phthalazin-1-ylacetate (1.29 g), 2-amino-4,6-difluoro-thiophenol hydrochloride (0.98 g) and ethanol (20 ml) was refluxed for 6 hours. Upon cooling, the title compound precipitated out as a pale yellow solid (yield: 1.62 g; m.p. 115-117°C).

### Example 16

### 3-(5-Trifluoromethylbenzothiazole-2-ylmethyl)-4-oxo-3-H-phthalazin-1-ylacetic acid

### Procedure A

A mixture of ethyl-3-(5-trifluoromethylbenzothiazole-2-ylmethyl-4-oxo-3-H-phthalazin-1-ylacetate (5.0 g), methanol (60 ml), tetrahydrofuran (30 ml) and 10% aqueous potassium hydroxide (20 ml) was stirred for 10 minutes at room temperature. The solution was concentrated to a volume of 20 ml and was then diluted with water (50 ml). The resulting solution was acidified to pH about 4.0 by addition of sufficient 10% HCl. The precipitated off-white solid was collected and air-dried (yield: 4.8 g; m.p. 197-198°C).

### Procedure B

A mixture of ethyl-3-cyanomethyl-4-oxo-phthalazin-1-ylacetate (2.71 g), 2-amino-4-trifluoromethyl-thiophenol hydrochloride (2.40 g) and ethanol (20 ml) was refluxed overnight. The reaction mixture was cooled to 40°C and to it was added tetrahydrofuran (10 ml) and 5% aqueous potassium hydroxide (10 ml). The mixture was stirred for 1 hour at room temperature and the solvents were removed by evaporation. The residue was diluted with water (50 ml) and the resulting solution was extracted with ether (20 ml). The basic aqueous layer was collected and acidified to pH of about 4.0 by addition of sufficient 10 % HCl. The precipitated solid was collected and air-dried (yield: 3.68 g; m.p. 197-198°C).

### Example 17

### 3-(5,7-Difluorobenzothiazole-2-ylmethyl)-4-oxo-3-H-phthalazin-1-ylacetic acid

Ethyl 3-(5,7-difluorobenzothiazol-2-ylmethyl)-4-oxo-3-H-phthalazin-1-ylacetate (1.0 g) was hydrolyzed according to the method of Example 16, Procedure A, to obtain the title compound (m.p. 178°C).

### Example 18

### 3-(5-Trifluoromethylbenzothiazole-2-ylethyl)-4-oxo-3-H-phthalazin-1-ylacetic acid

A mixture of ethyl-3-cyanoethyl-4-oxo-3-H-phthalazin-1-ylacetate (2.71 g), 2-amino-4-trifluoromethyl-thiophenol hydrochloride (2.29 g) and ethanol (20 ml) was refluxed overnight. The crude product obtained upon evaporation of ethanol was purified by chromatography on silica gel, eluting with a mixture of hexane and tetrahydrofuran (4:1). The white solid obtained (0.3 g) was used directly in the next step which consisted of dissolving the compound in ethanol (20 ml) containing 5% aqueous KOH (2 ml) and stirring at room temperature for 2 hours. The ethanol was evaporated, the residue was diluted with water (10 ml), extracted with ether (2 x 10 ml) and the aqueous extract acidified to pH 2.0. The precipitated solid was collected and then crystallized from ethanol (yield: 0.12 g; m.p. 184-185°C).

### Example 19

### Ethyl-3-(ethyl acetimidate)-4-oxo-3H-phthalazin-1-ylacetate, hydrochloride

Dry hydrogen chloride gas was passed for 5 minutes into a solution of 3-cyanomethyl-4-oxo-phthalazin-1-ylacetate (27.1 g) in dry tetrahydrofuran (200 ml) and absolute ethanol (5.9 ml). The precipitate formed upon letting the reaction stand at room temperature overnight was filtered off to obtain the desired product (13.05 g, m.p. 208-210°C).

### Example 20

### Ethyl-3-(5-trifluoromethylbenzothiazole-2-ylmethyl)-4-oxo-3H-phthalazin-1-ylacetate

A mixture of ethyl-3-ethyl acetimidate)-4-oxo-3H-phthalazin-1-ylacetate hydrochloride (0.35 g) and 2-amino-5-trifluoromethyl thiophenol hydrochloride (0.23 g) in toluene (20 ml) was refluxed for 16 hours. The precipitate obtained upon cooling was crystallized from ethanol to obtain the product (m.p. 136°C).

### Example 21

### Ethyl-3-thiocyanatomethyl-4-oxo-3-H-phthalazin-1-ylacetate

Following the method of Procedure D of Example 7, but replacing potassium cyanide by potassium thiocyanate, prepare the title compound.

## Claims (Claims for the following Contracting State(s): AT, BE, CH, DE, FR, GB, GR, IT, LI, LU, NL, SE)

1. A process for the preparation of a compound of the formula 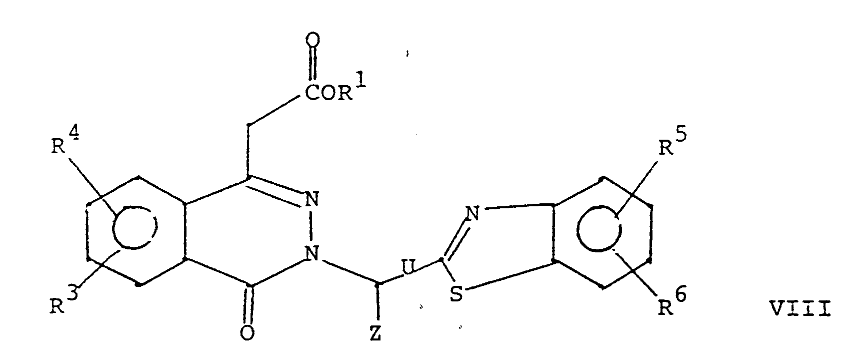 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; R⁵ and R⁶ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, nitro, or benzo or R⁵ and R⁶ taken together are C₁-C₄ alkanedioxy; Z is hydrogen or methyl; and U is S, CH₂ or a covalent bond, comprising reacting a compound of the formula 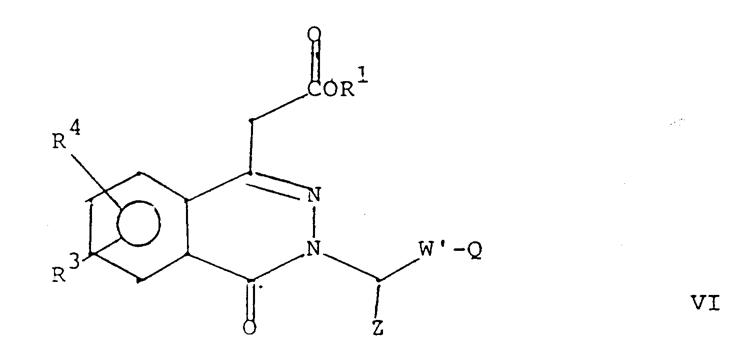 wherein R¹, R³, R⁴ and Z are as defined above, W' is S, CH₂ or a covalent bond, and Q is -CN or 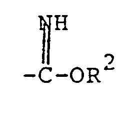 wherein R² is C₁-C₄ alkyl, or an acid addition thereof with a compound of the formula 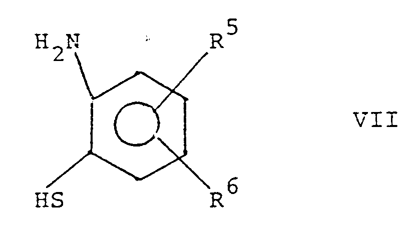 wherein R⁵ and R⁶ are defined above, or with an acid addition salt thereof, with the proviso that when neither one of said compounds of the formula VI and the formula VII is in the form of its acid addition salt when it is added to the other of said compounds, the reaction is conducted in the presence of a strong acid; and, if desired, hydrolyzing a compound of the formula VIII wherein R¹ is other than hydrogen to the corresponding compound of the formula VIII wherein R¹ is hydrogen.

2. A process according to claim 1, wherein said compound of the formula VI wherein Q is -CN is prepared by reacting a compound of the formula 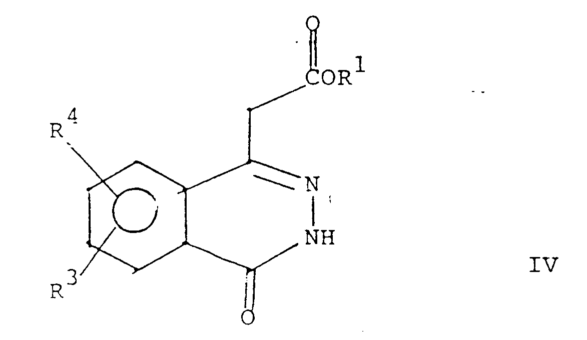 wherein R¹, R³ and R⁴ are as defined in claim 1, with a compound of the formula 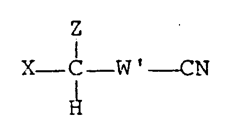 wherein X is chloro, bromo, -OSO₂-(C₁ to C₄ alkyl), or -OSO₂-aryl wherein aryl is phenyl, naphthyl, substituted phenyl, or substituted naphthyl, wherein the substituents on the substituted phenyl and substituted naphthyl groups are C₁ to C₄ alkyl, halogen or nitro, in the presence of a suitable base.

3. A process according to claim 1, wherein a compound of the formula VI wherein W' is a covalent bond and Z is hydrogen is prepared by reacting a compound of the formula 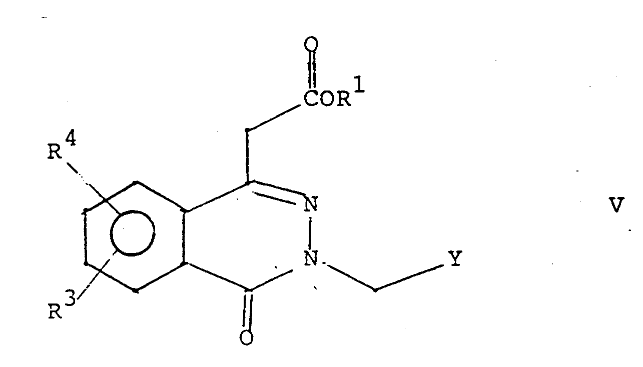 wherein Y is chloro or bromo and R¹, R³ and R⁴ are as defined for formula V in claim 1, with an alkali metal cyanide or alkaline earth metal cyanide.

4. A process according to claim 1, wherein a compound of the formula VI is co-melted with a preformed acid addition salt of a compound of the formula VII at a temperature between about 110 and about 180°C.

5. A process for the preparation of a compound of the formula 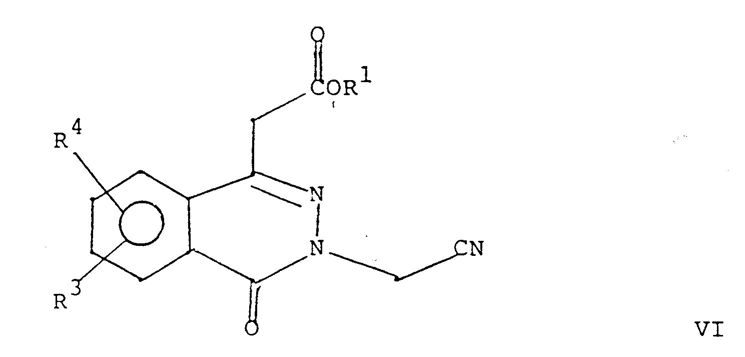 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy, comprising reacting a compound of the formula 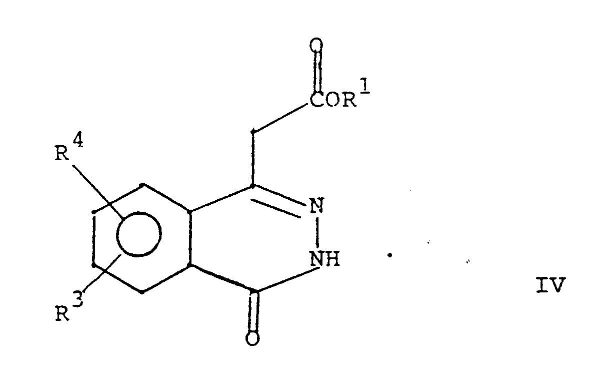 wherein R¹, R³ and R⁴ are as defined above with a compound of the formula XCH₂CN wherein X is chloro, bromo, -OSO₂-(C₁ to C₄ alkyl), or -OSO₂-aryl wherein aryl is phenyl, naphthyl, substituted phenyl, or substituted naphthyl, wherein the substituents on the substituted phenyl and substituted naphthyl groups are C₁ to C₄ alkyl, halogen or nitro; Z is hydrogen or methyl and W' is CH₂ or a covalent bond, in the presence of a suitable base.

6. A process for the preparation of a compound of the formula 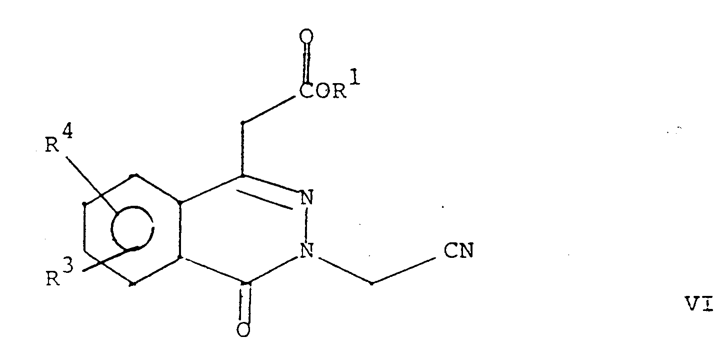 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy, comprising reacting a compound of the formula 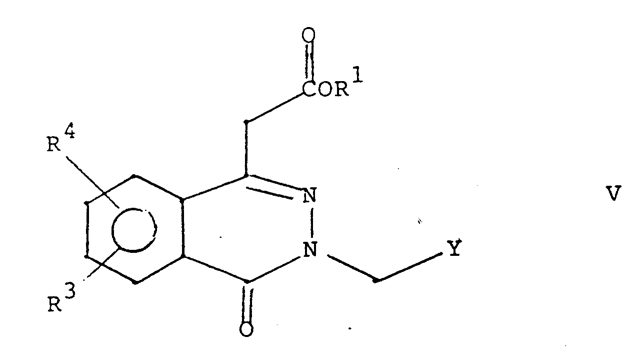 wherein Y is chloro or bromo and R¹, R³ and R⁴ are as defined above with alkali metal cyanide or alkaline earth metal cyanide.

7. A process for the preparation of a compound of the formula 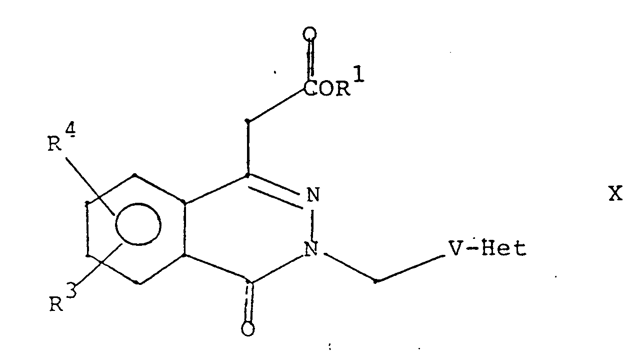 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; V is oxygen, sulfur or NH; and Het is a heterocyclic 5-membered ring having one nitrogen, oxygen or sulfur, two nitrogens one of which may be replaced by oxygen or sulfur, or three nitrogens one of which may be replayed by oxygen or sulfur, said ring substituted by one or two fluoro, chloro, C₁-C₄ or alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro; chloro, bromo, C₁-C₄ alkyl or C₁-C₄ alkoxy; a heterocyclic 6-membered ring having one to three nitrogen atoms, or one or two nitrogen atoms and one oxygen or sulfur, and said ring substituted by one or two C₁-C₄ alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro, chloro, C₁-C₄ alkyl or C₁-C₄ alkoxy; oxazole or thiazole condensed with a 6-membered aromatic group containing one or two nitrogen atoms or with thiophene or furane, each optionally substituted by one of fluoro, chloro, bromo, trifluoromethyl, methylthio or methylsulfinyl; imidazolopyridine; naphthothiazole; or naphthoxazole, comprising reacting a compound of the formula 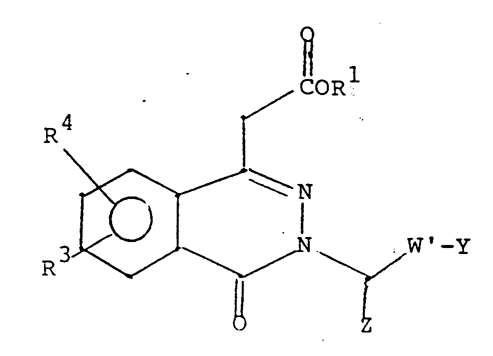 wherein R¹, R³ and R⁴ are as defined above, W' is a covalent bond, Z is H and Y is chloro or bromo, with a compound of the formula HV-Het wherein V and Het are as defined above.

8. A compound of the formula 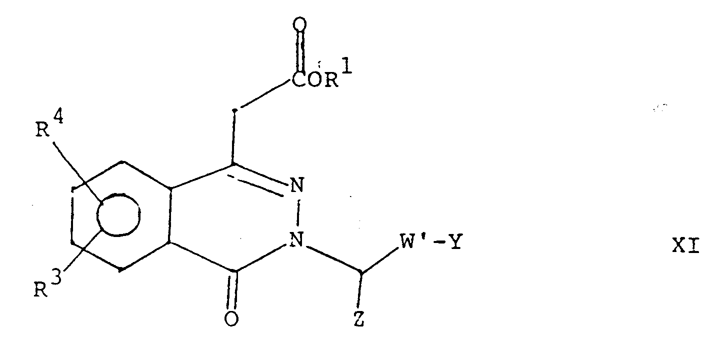 wherein R¹ is hydrogen or C₁ to C₄ alkyl; and R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; Z is hydrogen or methyl; W' is S, CH₂ or a covalent bond; and Y is hydroxy, chloro, bromo, cyano or 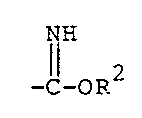 wherein R² is C₁-C₄ alkyl, with the proviso that when W' is S, Y cannot be hydroxy, chloro or bromo.

## Claims (Claims for the following Contracting State(s): ES)

1. A process for the preparation of a compound of the formula 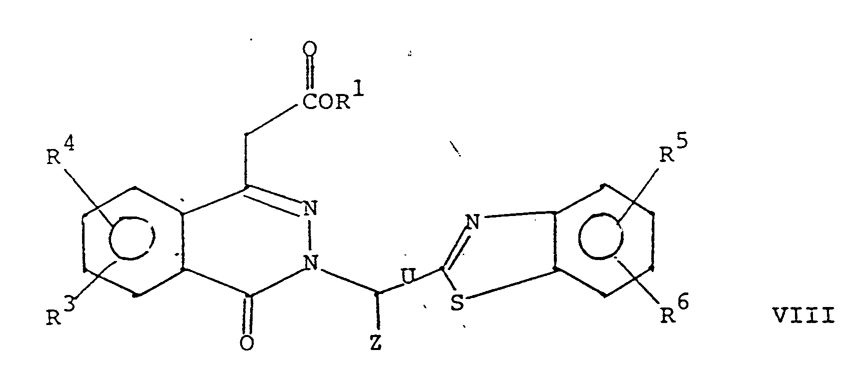 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; R⁵ and R⁶ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, nitro, or benzo or R⁵ and R⁶ taken together are C₁-C₄ alkanedioxy; Z is hydrogen or methyl; and U is S, CH₂ or a covalent bond, comprising reacting a compound of the formula 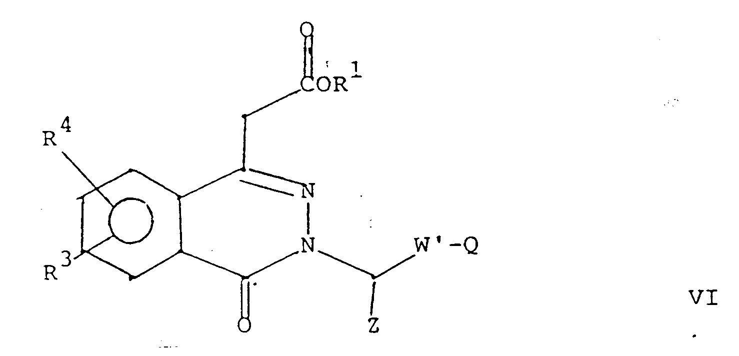 wherein R¹, R³, R⁴ and Z are as defined above, W' is S, CH₂ or a covalent bond, and Q is -CN or 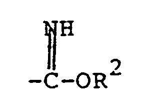 wherein R² is C₁-C₄ alkyl, or an acid addition thereof with a compound of the formula 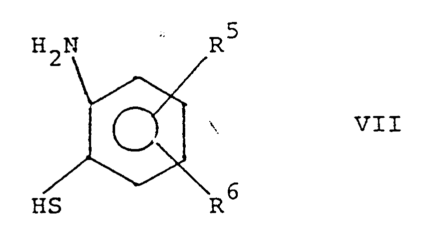 wherein R⁵ and R⁶ are defined above, or with an acid addition salt thereof, with the proviso that when neither one of said compounds of the formula VI and the formula VII is in the form of its acid addition salt when it is added to the other of said compounds, the reaction is conducted in the presence of a strong acid; and, if desired, hydrolyzing a compound of the formula VIII wherein R¹ is other than hydrogen to the corresponding compound of the formula VIII wherein R¹ is hydrogen.

2. A process according to claim 1, wherein said compound of the formula VI wherein Q is -CN is prepared by reacting a compound of the formula 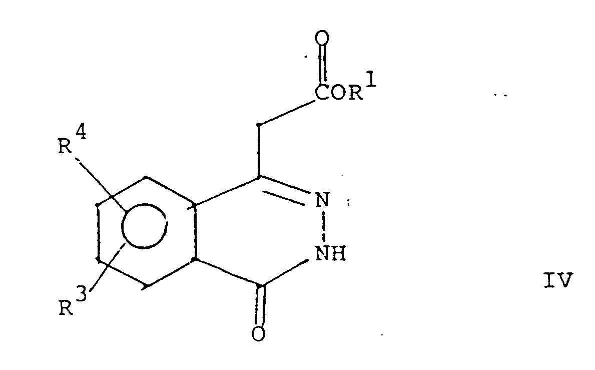 wherein R¹, R³ and R⁴ are as defined in claim 1, with a compound of the formula 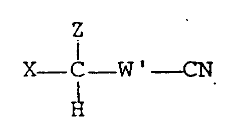 wherein X is chloro, bromo, -OSO₂-(C₁ to C₄ alkyl), or -OSO₂-aryl wherein aryl is phenyl, naphthyl, substituted phenyl, or substituted naphthyl, wherein the substituents on the substituted phenyl and substituted naphthyl groups are C₁ to C₄ alkyl, halogen or nitro, in the presence of a suitable base.

3. A process according to claim 1, wherein a compound of the formula VI wherein W' is a covalent bond and Z is hydrogen is prepared by reacting a compound of the formula 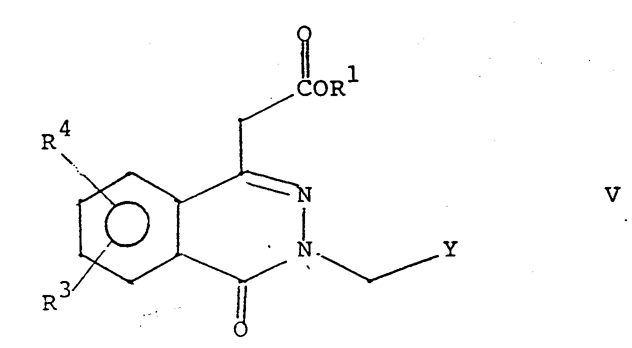 wherein Y is chloro or bromo and R¹, R³ and R⁴ are as defined for formula V in claim 1, with an alkali metal cyanide or alkaline earth metal cyanide.

4. A process according to claim 1, wherein a compound of the formula VI is co-melted with a preformed acid addition salt of a compound of the formula VII at a temperature between about 110 and about 180°C.

5. A process for the preparation of a compound of the formula 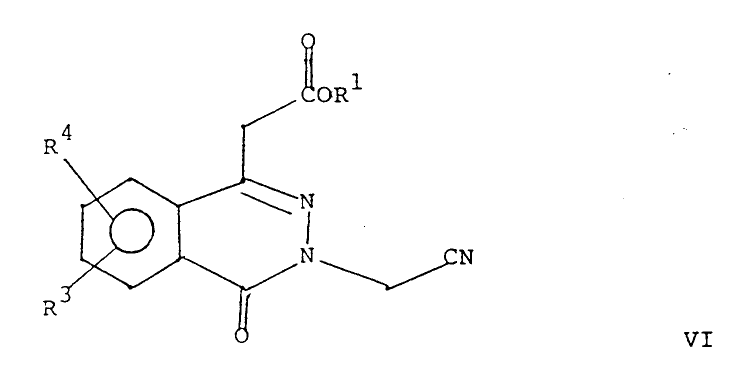 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy, comprising reacting a compound of the formula 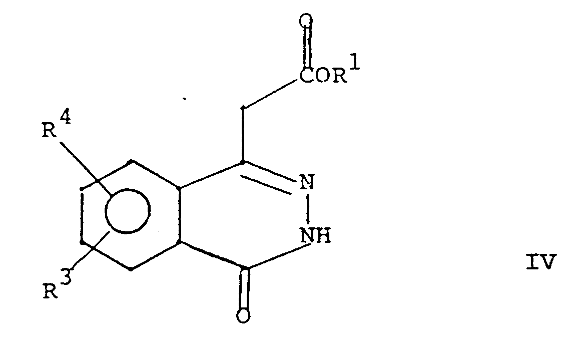 wherein R¹, R³ and R⁴ are as defined above with a compound of the formula XCH₂CN wherein X is chloro, bromo, -OSO₂-(C₁ to C₄ alkyl), or -OSO₂-aryl wherein aryl is phenyl, naphthyl, substituted phenyl, or substituted naphthyl, wherein the substituents on the substituted phenyl and substituted naphthyl groups are C₁ to C₄ alkyl, halogen or nitro; Z is hydrogen or methyl and W' is CH₂ or a covalent bond, in the presence of a suitable base.

6. A process for the preparation of a compound of the formula 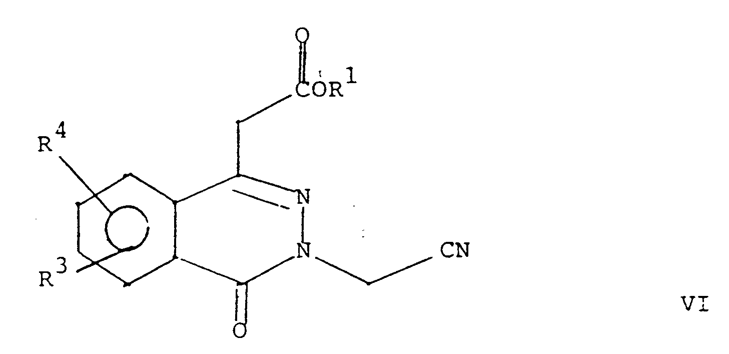 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy, comprising reacting a compound of the formula 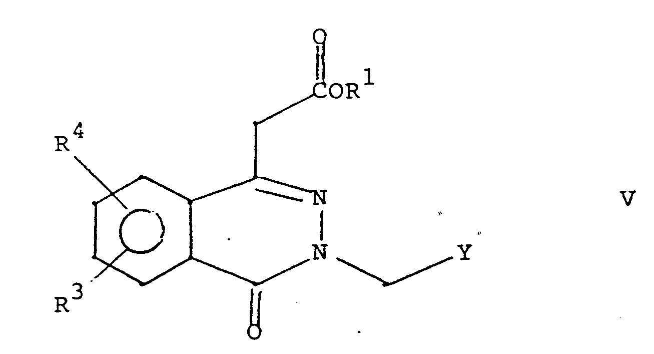 wherein Y is chloro or bromo and R¹, R³ and R⁴ are as defined above with alkali metal cyanide or alkaline earth metal cyanide.

7. A process for the preparation of a compound of the formula 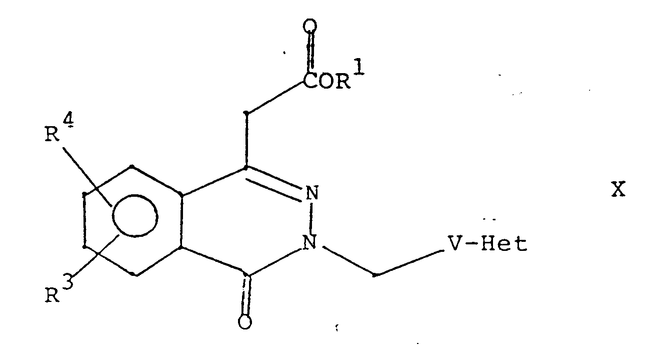 wherein R¹ is hydrogen or C₁ to C₄ alkyl; R³ and R⁴ are the same or different and are hydrogen, fluoro, chloro, bromo, trifluoromethyl, C₁-C₄ alkyl, C₁-C₄ alkoxy, C₁-C₄ alkylthio, C₁-C₄ alkylsulfinyl, C₁-C₄ alkylsulfonyl, or nitro, or R³ and R⁴ taken together are C₁-C₄ alkanedioxy; V is oxygen, sulfur or NH; and Het is a heterocyclic 5-membered ring having one nitrogen, oxygen or sulfur, two nitrogens one of which may be replaced by oxygen or sulfur, or three nitrogens one of which may be replayed by oxygen or sulfur, said ring substituted by one or two fluoro, chloro, C₁-C₄ or alkyl or phenyl, or condensed with benzo, or substituted by one of pyridyl, furyl or thienyl, said phenyl or benzo optionally substituted by one of iodo or trifluoromethylthio, or one for two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro, chloro, bromo, C₁-C₄ alkyl or C₁-C₄ alkoxy; a heterocyclic 6-membered ring having one to three nitrogen atoms, or one or two nitrogen atoms and one oxygen or sulfur, and said ring substituted by one or two C₁-C₄ alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, C₁-C₄ alkyl, C₁-C₄ alkoxy, C₁-C₄ alkylthio, C₁-C₄ alkylsulfinyl, C₁-C₄ alkylsulfonyl, or trifluoromethyl, and said pyridyl, furyl or thienyl optionally substituted in the 3-position by fluoro, chloro, C₁-C₄ alkyl or C₁-C₄ alkoxy; oxazole or thiazole condensed with a 6-membered aromatic group containing one or two nitrogen atoms or with thiophene or furane, each optionally substituted by one of fluoro, chloro, bromo, trifluoromethyl, methylthio or methylsulfinyl; imidazolopyridine; naphthothiazole; or naphthoxazole, comprising reacting a compound of the formula 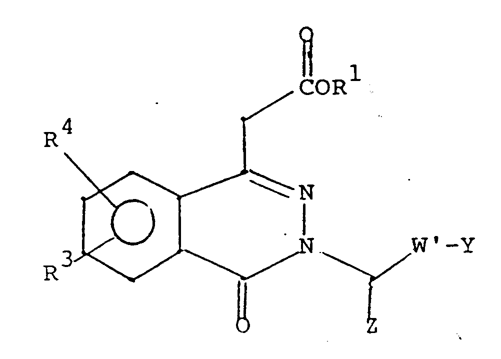 wherein R¹, R³ and R⁴ are as defined above, W' is a covalent bond, Z is H and Y is chloro or bromo, with a compound of the formula HV-Het wherein V and Het are as defined above.

## Patentansprüche (Patentansprüche für folgende(n) Vertragsstaat(en): AT, BE, CH, DE, FR, GB, GR, IT, LI, LU, NL, SE)

1. Verfahren zum Herstellen einer Verbindung mit der Formel 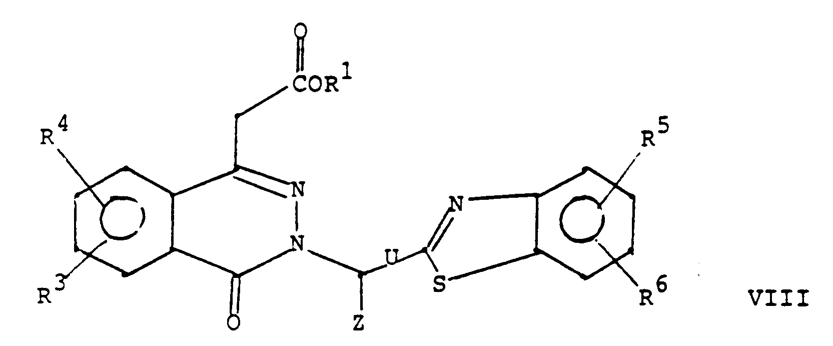 worin R¹ Wasserstoff oder C₁- bis C₄-Alkyl ist, R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten, R⁵ und R⁶ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, Nitro oder Benzo bedeuten oder R⁵ und R⁶ zusammengenommen C₁-C₄-Alkandioxy bedeuten, Z Wasserstoff oder Methyl ist und U S, CH₂ oder eine kovalente Bindung bedeutet, welches das Umsetzen einer Verbindung mit der Formel 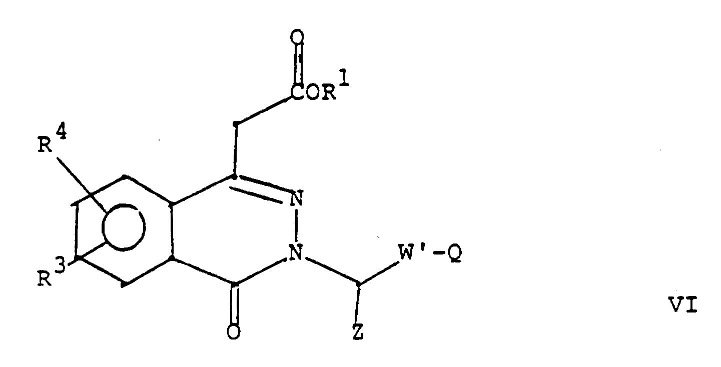 worin R¹, R³, R⁴ und Z wie oben definiert sind, W' S, CH₂ oder eine kovalente Bindung bedeutet und Q -CN oder 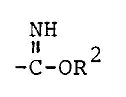 ist, worin R² C₁-C₄-Alkyl bedeutet,
oder eines Säure-Additionssalzes davon mit einer Verbindung mit der Formel 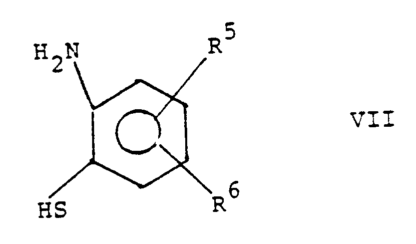 worin R⁵ und R⁶ wie oben definiert sind, oder mit einem Säure-Additionssalz davon
unter der Bedingung, daß, wenn keine der beiden Verbindungen mit der Formel VI und der Formel VII in Form ihres Säure-Additionssalzes vorliegt, wenn sie der anderen der beiden Verbindungen zugesetzt wird, die Umsetzung in Gegenwart einer starken Säure durchgeführt wird,
und, auf Wunsch, das Hydrolysieren der Verbindung mit der Formel VIII, worin R¹ nicht Wasserstoff bedeutet, zu der entsprechenden Verbindung mit der Formel VIII, worin R¹ Wasserstoff ist,
umfaßt.

2. Verfahren nach Anspruch 1, worin die Verbindung mit der Formel VI, worin Q -CN bedeutet, durch Umsetzen einer Verbindung mit der Formel 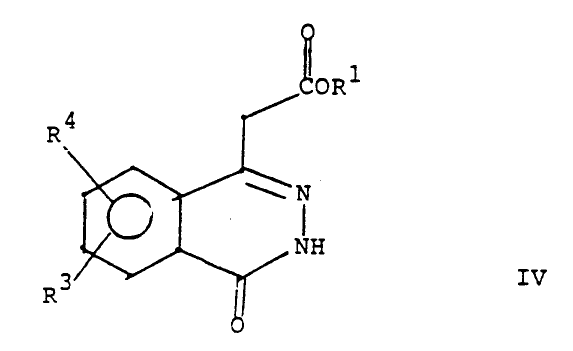 worin R¹, R³ und R⁴ wie in Anspruch 1 definiert sind, mit einer Verbindung mit der Formel 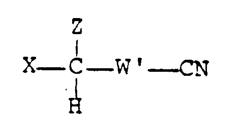 worin X Chlor, Brom, -OSO₂-(C₁- bis C₄-Alkyl) oder OSO₂-Aryl bedeutet, worin Aryl Phenyl, Naphthyl, substituiertes Phenyl oder substituiertes Naphthyl ist und wobei die Substituenten an den substituierten Phenyl- und den substituierten Naphthyl-Gruppen C₁- bis C₄-Alkyl, Halogen oder Nitro sind, in Gegenwart einer geeigneten Base hergestellt ist.

3. Verfahren nach Anspruch 1, worin die Verbindung mit der Formel VI, worin W' eine kovalente Bindung bedeutet und Z Wasserstoff ist, durch Umsetzen einer Verbindung mit der Formel 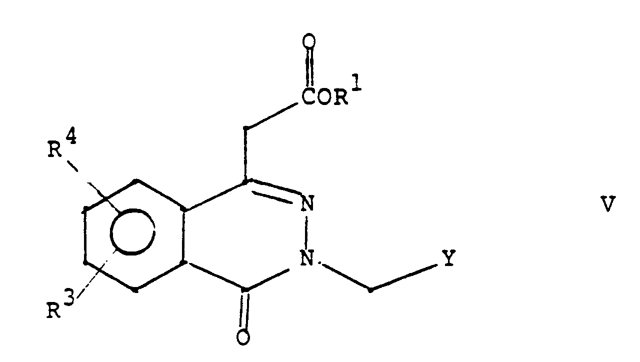 worin Y Chlor oder Brom ist und R¹, R³ und R⁴ wie für Formel V in Anspruch 1 definiert sind, mit einem Alkalimetallcyanid oder einem Erdalkalimetallcyanid hergestellt ist.

4. Verfahren nach Anspruch 1, worin eine Verbindung mit der Formel VI mit einem vorgebildeten Säure-Additionssalz einer Verbindung mit der Formel VII bei einer Temperatur zwischen etwa 110°C und etwa 180°C zusammengeschmolzen wird.

5. Verfahren zum Herstellen einer Verbindung mit der Formel 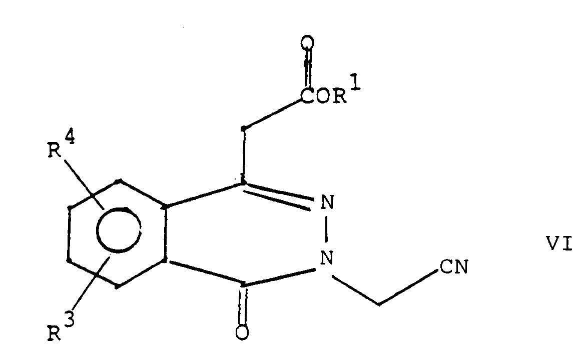 worin R¹ Wasserstoff oder C₁- bis C₄-Alkyl ist und R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro sind oder R³ und R⁴ zusammengenommen C₁-C₄-Alkandioxy bedeuten,
welches das Umsetzen einer Verbindung mit der Formel 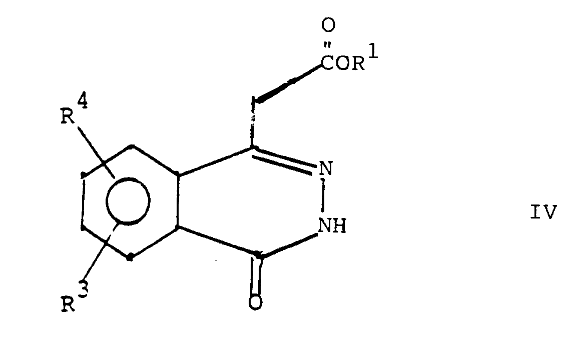 worin R¹, R³ und R⁴ wie oben definiert sind, mit einer Verbindung der Formel XCH₂CN, worin X Chlor, Brom, -OSO₂-(C₁-C₄-Alkyl) oder -OSO₂-Aryl ist, worin Aryl Phenyl, Naphthyl, substituiertes Phenyl oder substituiertes Naphthyl ist, worin die Substituenten an den substituierten Phenyl- und substituierten Naphthylgruppen C₁-C₄-Alkyl, Halogen oder Nitro sind, Z Wasserstoff oder Methyl ist und W' CH₂ oder eine kovalente Bindung ist, in Gegenwart einer geeigneten Base umfaßt.

6. Verfahren zum Herstellen einer Verbindung mit der Formel 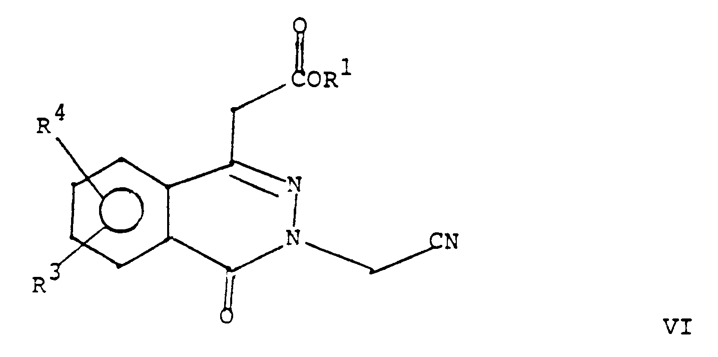 worin R¹ Wasserstoff oder C₁- bis C₄-Alkyl ist, R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten, welches das Umsetzen einer Verbindung mit der Formel 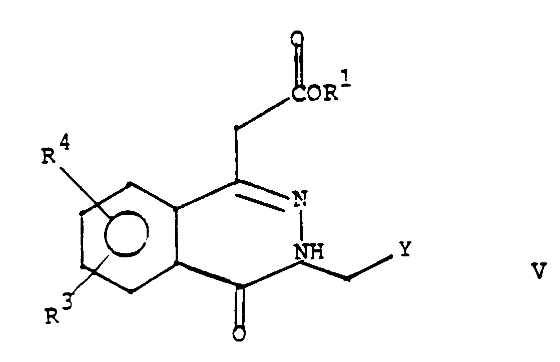 worin Y Chlor oder Brom ist und R¹, R³ und R⁴ wie oben definiert ist, mit einem Alkalimetallcyanid oder Erdalkalimetallcyanid umfaßt.

7. Verfahren zum Herstellen einer Verbindung mit der Formel 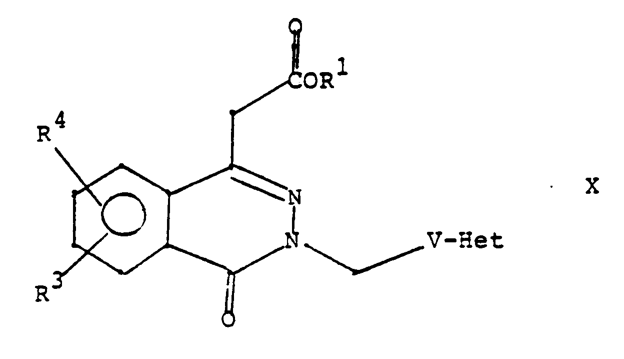 worin
R¹ Wasserstoff oder C₁- bis C₄-Alkyl ist,
R³ und R⁴ identisch oder unterschiedlich sind und
Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten,
V Sauerstoff, Schwefel oder NH ist und
Het ein heterocyclischer 5-gliedriger Ring mit einem Stickstoff, Sauerstoff oder Schwefel, zwei Stickstoffatomen, von denen eines durch Sauerstoff oder Schwefel ersetzt sein kann, oder drei Stickstoffatomen, von denen eines durch Sauerstoff oder Schwefel ersetzt sein kann, wobei dieser Ring mit einem oder zwei Fluor, Chlor, C₁-C₄-Alkyl oder Phenyl substituiert ist oder mit einer Benzogruppe kondensiert ist oder mit einer der Gruppen Pyridyl, Furyl oder Thienyl substituiert ist, wobei die Phenyl- oder die Benzogruppe fakultativ mit entweder einem Iod oder einem Trifluormethylthio oder einem oder zwei Fluor, Chlor, Brom, C₁-C₄-Alkyl, C₁-C₄-Alkoxy, C₁-C₄-Alkylthio, C₁-C₄-Alkylsulfinyl, C₁-C₄-Alkylsulfonyl oder Trifluormethyl substituiert ist und die Pyridyl-, Furyl- oder Thienylgruppe fakultativ in 3-Position durch Fluor, Chlor, Brom, C₁-C₄-Alkyl oder C₁-C₄-Alkoxy substituiert ist, ein heterocyclischer 6-gliedriger Ring, der ein bis drei Stickstoffatome oder ein oder zwei Stickstoffatome und ein Sauerstoff- oder Schwefelatom enthält, und wobei dieser Ring durch ein oder zwei C₁-C₄-Alkyl- oder Phenylgruppen substituiert oder mit einer Benzogruppe kondensiert ist oder mit einer der Gruppen Pyridyl, Furyl oder Thienyl substituiert ist, wobei die Phenyl- oder die Benzogruppe fakultativ mit entweder einem Iod oder einem Trifluormethylthio oder einem oder zwei Fluor, Chlor, Brom, C₁-C₄-Alkyl, C₁-C₄-Alkoxy, C₁-C₄-Alkylthio, C₁-C₄-Alkylsulfinyl, C₁-C₄-Alkylsulfonyl oder Trifluormethyl substituiert ist und die Pyridyl-, Furyl- oder Thienylgruppe fakultativ in 3-Position durch Fluor, Chlor, C₁-C₄-Alkyl oder C₁-C₄-Alkoxy substituiert ist, Oxazol oder Thiazol, kondensiert mit einer 6-gliedrigen aromatischen Gruppe, welche ein oder zwei Stickstoffatome enthält, oder mit Thiophen oder Furan, die jeweils fakultativ durch ein Fluor, Chlor, Brom, Trifluormethyl, Methylthio oder Methylsulfinyl substituiert sind, Imidazolpyridin, Naphthothiazol oder Naphthoxazol ist,
welches das Umsetzen einer Verbindung mit der Formel 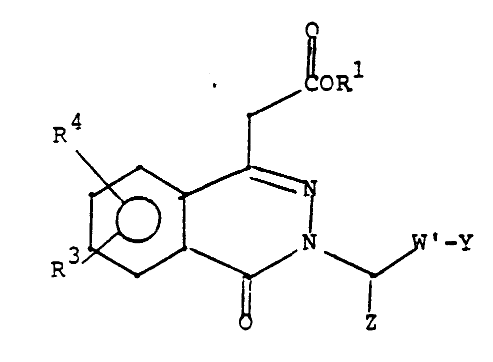 worin R¹, R³ und R⁴ wie oben definiert sind, W' eine kovalente Bindung ist, Z H ist und Y Chlor oder Brom ist, mit einer Verbindung der Formel HV-Het, worin V und Het wie oben definiert sind, umfaßt.

8. Verbindung mit der Formel 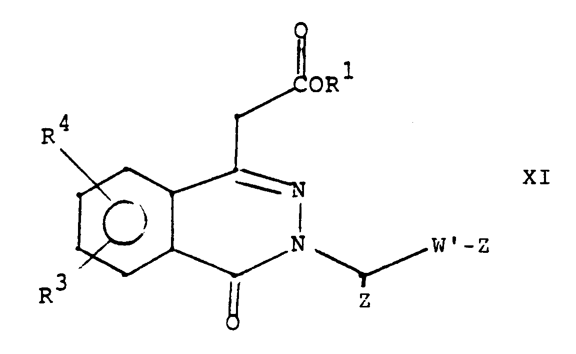 worin R¹ Wasserstoff oder C₁-C₄-Alkyl bedeutet und R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten, Z Wasserstoff oder Methyl ist, W' S CH₂ oder eine kovalente Bindung ist
und Y Hydroxy, Chlor, Borm, Cyano oder 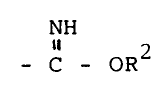 bedeutet, worin R² C₁-C₄-Alkyl ist, mit der Maßgabe, daß, wenn W' S ist, Y nicht Hydroxy, Chlor oder Brom sein kann.

## Patentansprüche (Patentansprüche für folgende(n) Vertragsstaat(en): ES)

1. Verfahren zum Herstellen einer Verbindung mit der Formel 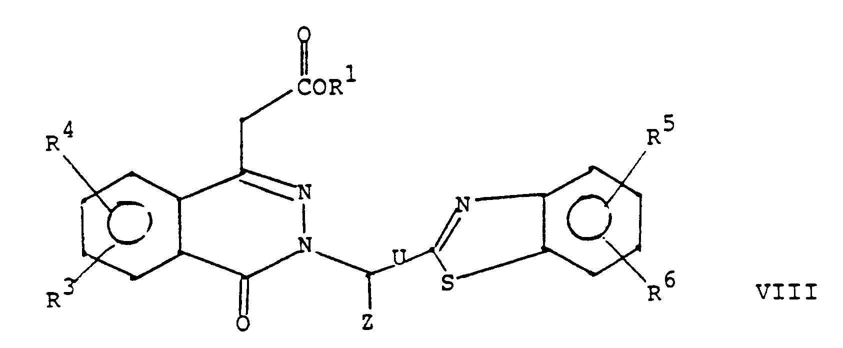 worin R¹ Wasserstoff oder C₁- bis C₄-Alkyl ist, R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten, R⁵ und R⁶ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, Nitro oder Benzo bedeuten oder R⁵ und R⁶ zusammengenommen C₁-C₄-Alkandioxy bedeuten, Z Wasserstoff oder Methyl ist und U S, CH₂ oder eine kovalente Bindung bedeutet, welches das Umsetzen einer Verbindung mit der Formel 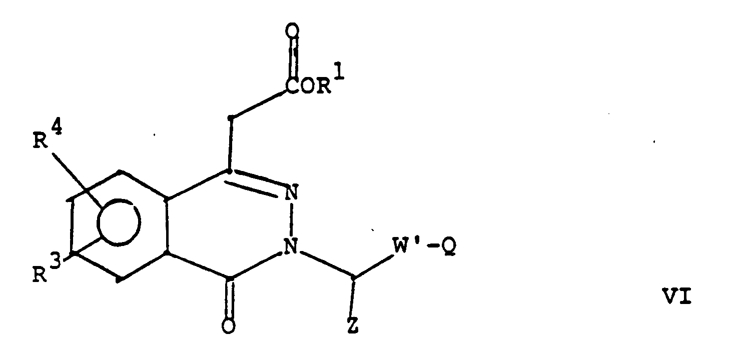 worin R¹, R³, R⁴ und Z wie oben definiert sind, W' S, CH₂ oder eine kovalente Bindung bedeutet und Q -CN oder 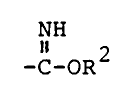 ist, worin R² C₁-C₄-Alkyl bedeutet,
oder eines Säure-Additionssalzes davon mit einer Verbindung mit der Formel 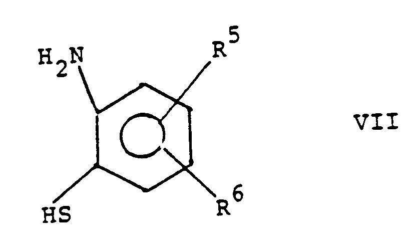 worin R⁵ und R⁶ wie oben definiert sind, oder mit einem Säure-Additionssalz davon
unter der Bedingung, daß, wenn keine der beiden Verbindungen mit der Formel VI und der Formel VII in Form ihres Säure-Additionssalzes vorliegt, wenn sie der anderen der beiden Verbindungen zugesetzt wird, die Umsetzung in Gegenwart einer starken Säure durchgeführt wird,
und, auf Wunsch, das Hydrolysieren der Verbindung mit der Formel VIII, worin R¹ nicht Wasserstoff bedeutet, zu der entsprechenden Verbindung mit der Formel VIII, worin R¹ Wasserstoff ist, umfaßt.

2. Verfahren nach Anspruch 1, worin die Verbindung mit der Formel VI, worin Q -CN bedeutet, durch Umsetzen einer Verbindung mit der Formel 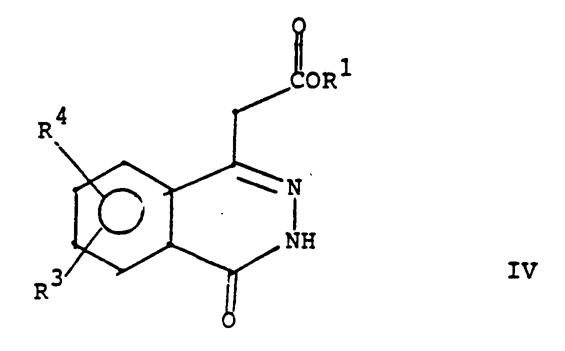 worin R¹, R³ und R⁴ wie in Anspruch 1 definiert sind, mit einer Verbindung mit der Formel 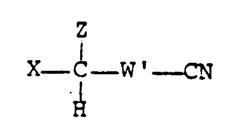 worin X Chlor, Brom, -OSO₂-(C₁- bis C₄-Alkyl) oder OSO₂-Aryl bedeutet, worin Aryl Phenyl, Naphthyl, substituiertes Phenyl oder substituiertes Naphthyl ist und wobei die Substituenten an den substituierten Phenyl- und den substituierten Naphthyl-Gruppen C₁- bis C₄-Alkyl, Halogen oder Nitro sind, in Gegenwart einer geeigneten Base hergestellt ist.

3. Verfahren nach Anspruch 1, worin die Verbindung mit der Formel VI, worin W' eine kovalente Bindung bedeutet und Z Wasserstoff ist, durch Umsetzen einer Verbindung mit der Formel 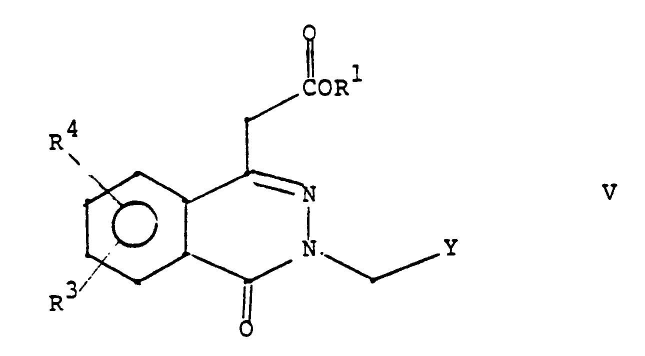 worin Y Chlor oder Brom ist und R¹, R³ und R⁴ wie für Formel V in Anspruch 1 definiert sind, mit einem Alkalimetallcyanid oder einem Erdalkalimetallcyanid hergestellt ist.

4. Verfahren nach Anspruch 1, worin eine Verbindung mit der Formel VI mit einem vorgebildeten Säure-Additionssalz einer Verbindung mit der Formel VII bei einer Temperatur zwischen etwa 110°C und etwa 180°C zusammengeschmolzen wird.

5. Verfahren zum Herstellen einer Verbindung mit der Formel 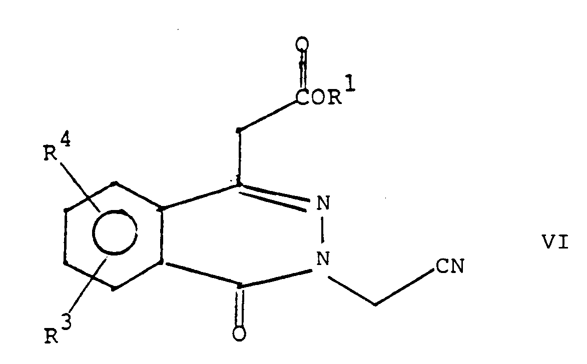 worin R¹ Wasserstoff oder C₁- bis C₄-Alkyl ist und R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro sind oder R³ und R⁴ zusammengenommen C₁-C₄-Alkandioxy bedeuten,
welches das Umsetzen einer Verbindung mit der Formel 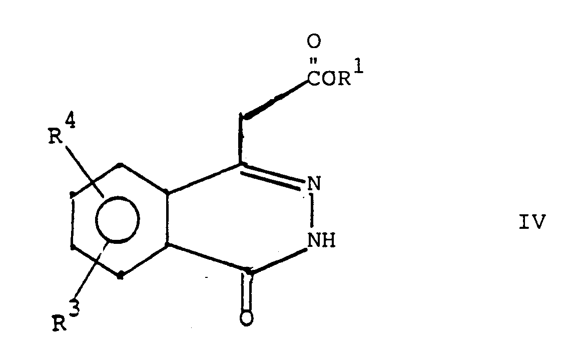 worin R¹, R³ und R⁴ wie oben definiert sind, mit einer Verbindung der Formel XCH₂CN, worin X Chlor, Brom, -OSO₂-(C₁-C₄-Alkyl) oder -OSO₂-Aryl ist, worin Aryl Phenyl, Naphthyl, substituiertes Phenyl oder substituiertes Naphthyl ist, worin die Substituenten an den substituierten Phenyl- und substituierten Naphthylgruppen C₁-C₄-Alkyl, Halogen oder Nitro sind, Z Wasserstoff oder Methyl ist und W' CH₂ oder eine kovalente Bindung ist, in Gegenwart einer geeigneten Base umfaßt.

6. Verfahren zum Herstellen einer Verbindung mit der Formel 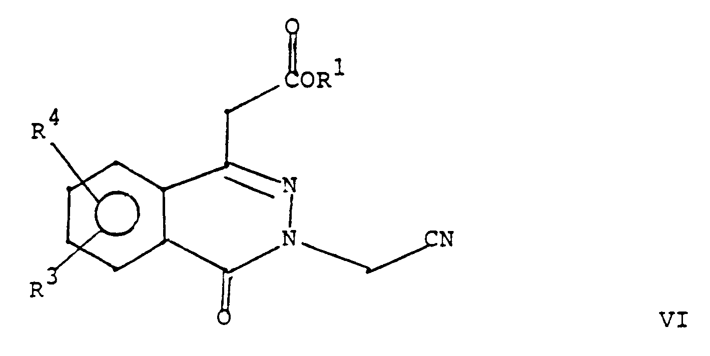 worin R¹ Wasserstoff oder C₁- bis C₄-Alkyl ist, R³ und R⁴ identisch oder unterschiedlich sind und Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten, welches das Umsetzen einer Verbindung mit der Formel 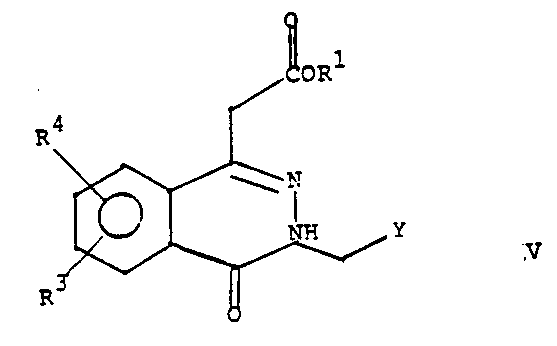 worin Y Chlor oder Brom ist und R¹, R³ und R⁴ wie oben definiert ist, mit einem Alkalimetallcyanid oder Erdalkalimetallcyanid umfaßt.

7. Verfahren zum Herstellen einer Verbindung mit der Formel 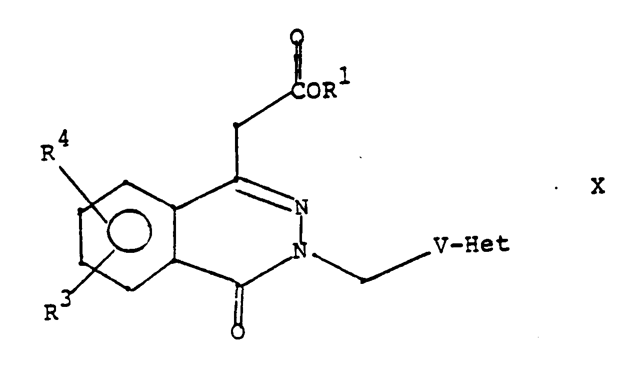 worin
R¹ Wasserstoff oder C₁- bis C₄-Alkyl ist,
R³ und R⁴ identisch oder unterschiedlich sind und
Wasserstoff, Fluor, Chlor, Brom, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl oder Nitro bedeuten oder R³ und R⁴ zusammengenommen C₁-C₄-Alkandioxy bedeuten,
V Sauerstoff, Schwefel oder NH ist und
Het ein heterocyclischer 5-gliedriger Ring mit einem Stickstoff, Sauerstoff oder Schwefel, zwei Stickstoffatomen, von denen eines durch Sauerstoff oder Schwefel ersetzt sein kann, oder drei Stickstoffatomen, von denen eines durch Sauerstoff oder Schwefel ersetzt sein kann, wobei dieser Ring mit einem oder zwei Fluor, Chlor, C₁-C₄-Alkyl oder Phenyl substituiert ist oder mit einer Benzogruppe kondensiert ist oder mit einer der Gruppen Pyridyl, Furyl oder Thienyl substituiert ist, wobei die Phenyl- oder die Benzogruppe fakultativ mit entweder einem Iod oder einem Trifluormethylthio oder einem oder zwei Fluor, Chlor, Brom, C₁-C₄-Alkyl, C₁-C₄-Alkoxy, C₁-C₄-Alkylthio, C₁-C₄-Alkylsulfinyl, C₁-C₄-Alkylsulfonyl oder Trifluormethyl substituiert ist und die Pyridyl-, Furyl- oder Thienylgruppe fakultativ in 3-Position durch Fluor, Chlor, Brom, C₁-C₄-Alkyl oder C₁-C₄-Alkoxy substituiert ist, ein heterocyclischer 6-gliedriger Ring, der ein bis drei Stickstoffatome oder ein oder zwei Stickstoffatome und ein Sauerstoff- oder Schwefelatom enthält, und wobei dieser Ring durch ein oder zwei C₁-C₄-Alkyl- oder Phenylgruppen substituiert oder mit einer Benzogruppe kondensiert ist oder mit einer der Gruppen Pyridyl, Furyl oder Thienyl substituiert ist, wobei die Phenyl- oder die Benzogruppe fakultativ mit entweder einem Iod oder einem Trifluormethylthio oder einem oder zwei Fluor, Chlor, Brom, C₁-C₄-Alkyl, C₁-C₄-Alkoxy, C₁-C₄-Alkylthio, C₁-C₄-Alkylsulfinyl, C₁-C₄-Alkylsulfonyl oder Trifluormethyl substituiert ist und die Pyridyl-, Furyl- oder Thienylgruppe fakultativ in 3-Position durch Fluor, Chlor, C₁-C₄-Alkyl oder C₁-C₄-Alkoxy substituiert ist, Oxazol oder Thiazol, kondensiert mit einer 6-gliedrigen aromatischen Gruppe, welche ein oder zwei Stickstoffatome enthält, oder mit Thiophen oder Furan, die jeweils fakultativ durch ein Fluor, Chlor, Brom, Trifluormethyl, Methylthio oder Methylsulfinyl substituiert sind, Imidazolpyridin, Naphthothiazol oder Naphthoxazol ist,
welches das Umsetzen einer Verbindung mit der Formel 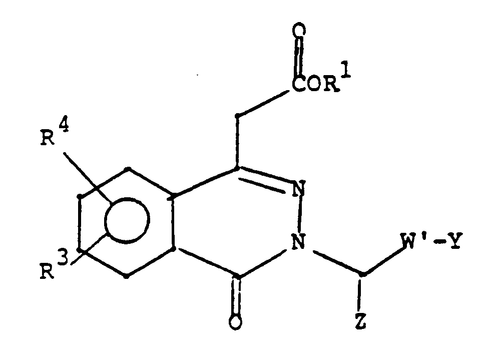 worin R¹, R³ und R⁴ wie oben definiert sind, W' eine kovalente Bindung ist, Z H ist und Y Chlor oder Brom ist, mit einer Verbindung der Formel HV-Het, worin V und Het wie oben definiert sind, umfaßt.

## Revendications (Revendications pour l'(les) Etat(s) contractant(s) suivant(s): AT, BE, CH, DE, FR, GB, GR, IT, LI, LU, NL, SE)

1. Procédé de préparation d'un composé de formule 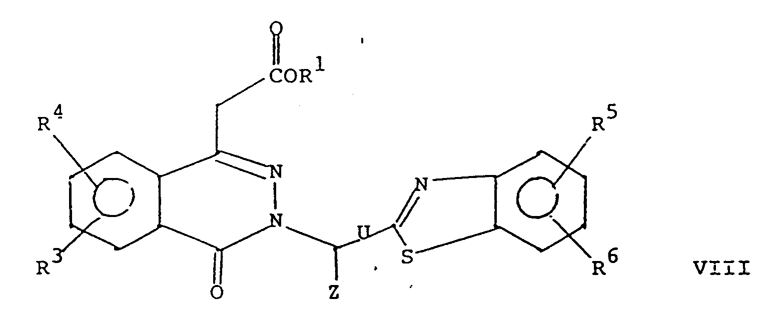 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄ ; R⁵ et R⁶ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄, nitro ou benzo, ou bien R⁵ et R⁶, pris conjointement, représentent des groupes alcanedioxy en C₁ à C₄ ; Z représente l'hydrogène ou un groupe méthyle ; et U représente S, un groupe CH₂ ou une liaison covalente, comprenant la réaction d'un composé de formule 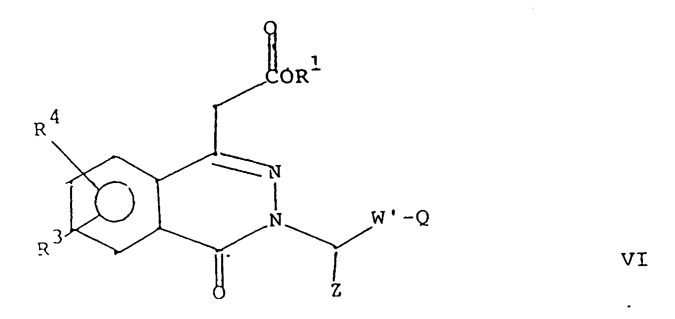 dans laquelle R¹, R³, R⁴ et Z répondent aux définitions précitées, W' représente S, un groupe CH₂ ou une liaison covalente, et Q représente un groupe -CN ou 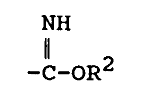 dans lequel R² représente un groupe alkyle en C₁ à C₄, ou d'un de ses sels d'addition d'acides, avec un composé de formule 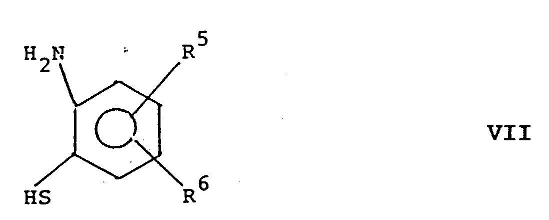 dans laquelle R⁵ et R⁶ répondent aux définitions précitées, ou avec un de ses sels d'addition d'acides, sous réserve que, lorsqu'aucun des composés de formule VI et de formule VII n'est sous forme de son sel d'addition d'acide lorsqu'il est ajouté à l'autre de ces composés, la réaction soit conduite en présence d'un acide fort ; et, si cela est désiré, l'hydrolyse d'un composé de formule VIII dans laquelle R¹ est autre que l'hydrogène en le composé correspondant de formule VIII dans laquelle R¹ représente l'hydrogène.

2. Procédé suivant la revendication 1, dans lequel le composé de formule VI dans laquelle Q représente un groupe -CN est préparé par réaction d'un composé de formule 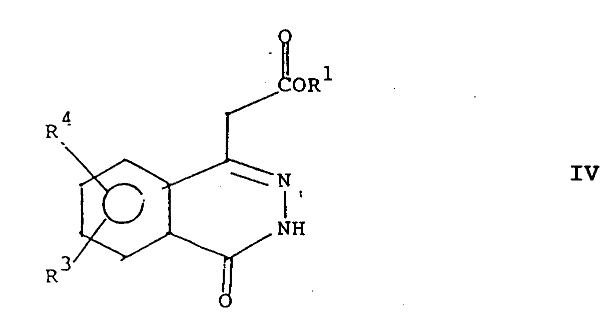 dans laquelle R¹, R³ et R⁴ répondent aux définitions suivant la revendication 1, avec un composé de formule 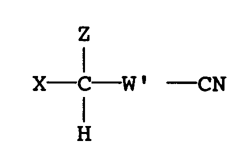 dans laquelle X représente un groupe chloro, bromo, -OSO₂-(alkyle en C₁ à C₄) ou -OSO₂-aryle dans lequel le groupe aryle est un groupe phényle, naphtyle, phényle substitué ou naphtyle substitué, les substituants présents sur les groupes phényle substitué et naphtyle substitué étant des groupes alkyle en C₁ à C₄, des halogènes ou des groupes nitro, en présence d'une base convenable.

3. Procédé suivant la revendication 1, dans lequel un composé de formule VI, dans laquelle W' représente une liaison covalente et Z représente l'hydrogène, est préparé par réaction d'un composé de formule 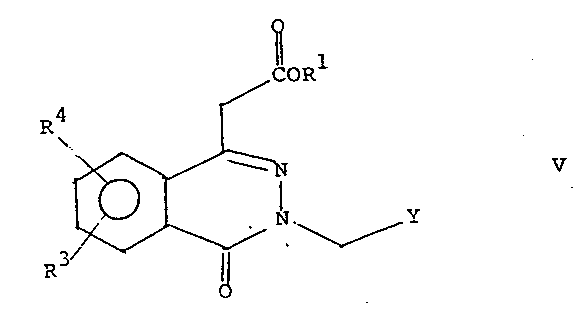 dans laquelle Y représente un groupe chloro ou bromo et R¹, R³ et R⁴ répondent aux définitions mentionnées pour la formule V dans la revendication 1, avec un cyanure de métal alcalin ou un cyanure de métal alcalino-terreux.

4. Procédé suivant la revendication 1, dans lequel un composé de formule VI est fondu conjointement avec un sel d'addition d'acide préformé d'un composé de formule VII à une température d'environ 110 à environ 180°C.

5. Procédé de préparation d'un composé de formule 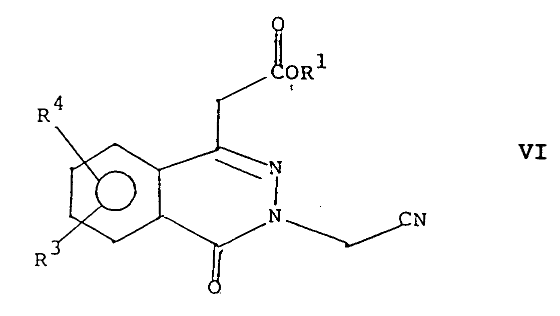 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄, comprenant la réaction d'un composé de formule 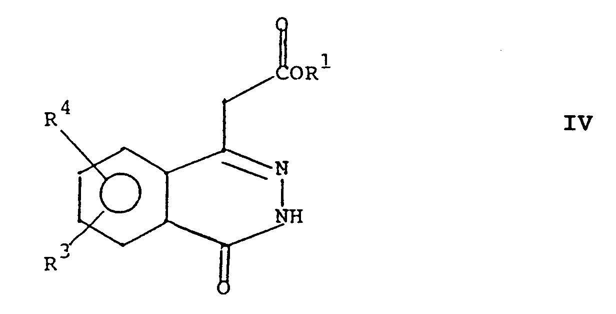 dans laquelle R¹, R³ et R⁴ répondent aux définitions précitées, avec un composé de formule XCH₂CN dans laquelle X représente un groupe chloro, bromo, -OSO₂-(alkyle en C₁ à C₄) ou -OSO₂-aryle dans lequel le groupe aryle est un groupe phényle, naphtyle, phényle substitué ou naphtyle substitué, les substituants présents sur le groupe phényle substitué et le groupe naphtyle substitué étant des groupes alkyle en C₁ à C₄, des halogènes ou des groupes nitro ; Z représente l'hydrogène ou un groupe méthyle et W' représente un groupe CH₂ ou une liaison covalente, en présence d'une base convenable.

6. Procédé de préparation d'un composé de formule 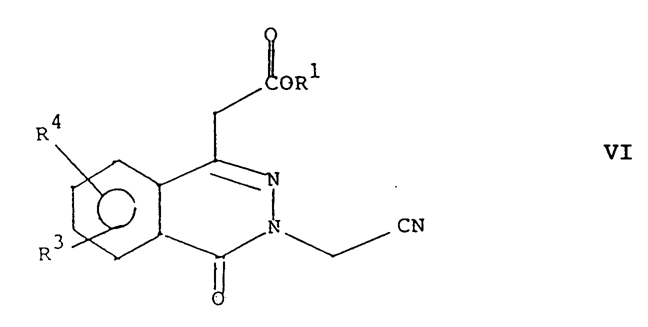 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄, comprenant la réaction d'un composé de formule 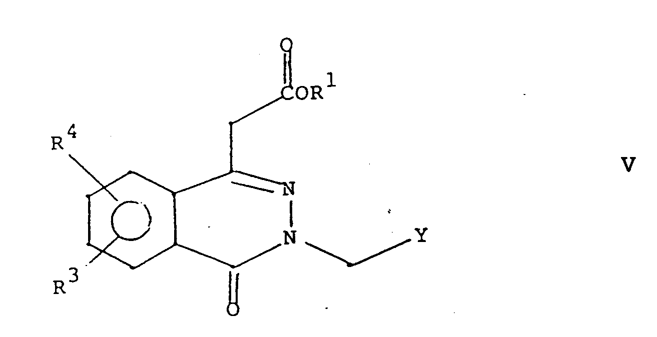 dans laquelle Y représente un groupe chloro ou bromo et R¹, R³ et R⁴ répondent aux définitions précitées, avec un cyanure de métal alcalin ou un cyanure de métal alcalino-terreux.

7. Procédé de préparation d'un composé de formule 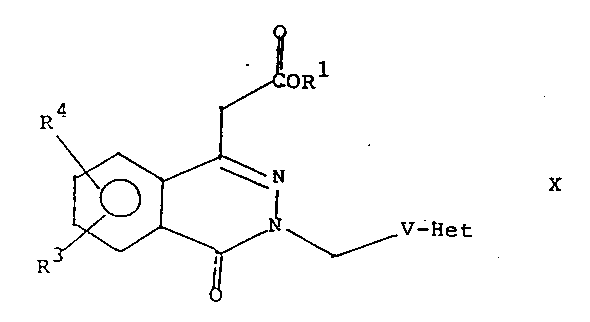 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄ ; V représente l'oxygène, le soufre ou un groupe NH ; et Het représente un noyau pentagonal hétérocyclique ayant un atome d'azote, d'oxygène ou de soufre, deux atomes d'azote dont l'un peut être remplacé par l'oxygène ou le soufre, ou trois atomes d'azote dont l'un peut être remplacé par l'oxygène ou le soufre, ledit noyau étant substitué avec un ou deux groupes fluoro, chloro, alkyle en C₁ à C₄ ou phényle, ou étant condensé avec un groupe benzo, ou étant substitué avec un groupe pyridyle, furyle ou thiényle, ledit groupe phényle ou benzo étant facultativement substitué avec un groupe iodo ou trifluorométhylthio, ou bien un ou deux groupes fluoro, chloro, bromo, alkyle en C₁ à C₄, alkoxy en C₁ à C₄, alkylthio en C₁ à C₄, alkylsulfinyle en C₁ à C₄, alkylsulfonyle en C₁ à C₄ ou trifluorométhyle, et ledit groupe pyridyle, furyle ou thiényle étant facultativement substitué en position 3 avec un groupe fluoro, chloro, bromo, alkyle en C₁ à C₄ ou alkoxy en C₁ à C₄ ; un noyau hexagonal hétérocyclique ayant 1 à 3 atomes d'azote, ou bien un ou deux atomes d'azote et un atome d'oxygène ou de soufre, ledit noyau étant substitué avec un ou deux groupes alkyle en C₁ à C₄ ou phényle, ou étant condensé avec un groupe benzo, ou étant substitué avec un groupe pyridyle, furyle ou thiényle, ledit groupe phényle ou benzo étant facultativement substitué avec un groupe iodo ou trifluorométhylthio, ou avec un ou deux groupes fluoro, chloro, bromo, alkyle en C₁ à C₄, alkoxy en C₁ à C₄, alkylthio en C₁ à C₄, alkylsulfinyle en C₁ à C₄, alkylsulfonyle en C₁ à C₄ ou trifluorométhyle, et ledit groupe pyridyle, furyle ou thiényle étant facultativement substitué en position 3 avec un groupe fluoro, chloro, alkyle en C₁ à C₄ ou alkoxy en C₁ à C₄ ; un groupe oxazole ou thiazole condensé avec un groupe aromatique hexagonal contenant un ou deux atomes d'azote ou avec le thiophène ou le furanne, chacun facultativement substitué avec un groupe fluoro, chloro, bromo, trifluorométhyle, méthylthio ou méthylsulfinyle ; imidazolopyridine ; naphtothiazole ; ou naphtoxazole, comprenant la réaction d'un composé de formule 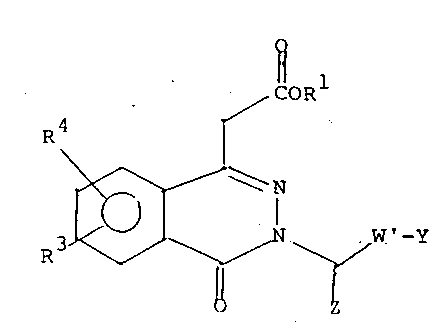 dans laquelle R¹, R³ et R⁴ répondent aux définitions précitées, W' représente une liaison covalente, Z représente H et Y représente un groupe chloro ou bromo, avec un composé de formule HV-Het dans laquelle V et Het répondent aux définitions précitées.

8. Composé de formule 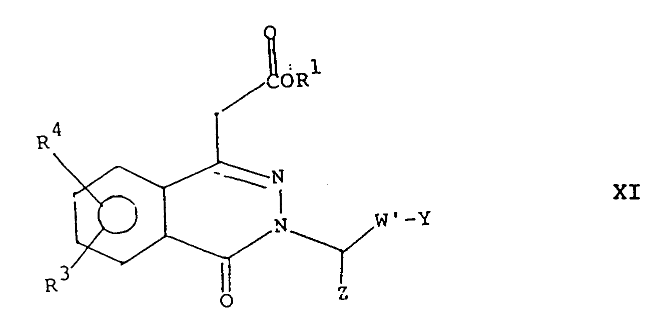 dans laquelle R¹ représente l'hydrogène ou un groupe alkyle en C₁ à C₄ ; et R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄ ; Z représente l'hydrogène ou un groupe méthyle ; W' représente S, un groupe CH₂ ou une liaison covalente ; et Y représente un groupe hydroxy, chloro, bromo, cyano ou 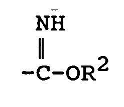 dans lequel R² représente un groupe alkyle en C₁ à C₄, sous réserve que, lorsque W' représente S, Y ne puisse représenter un groupe hydroxy, chloro ou bromo.

## Revendications (Revendications pour l'(les) Etat(s) contractant(s) suivant(s): ES)

1. Procédé de préparation d'un composé de formule 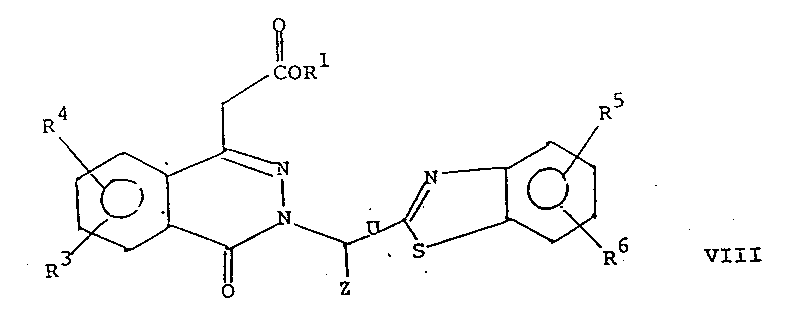 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄ ; R⁵ et R⁶ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄, nitro ou benzo, ou bien R⁵ et R⁶, pris conjointement, représentent un groupe alcanedioxy en C₁ à C₄ ; Z représente l'hydrogène ou un groupe méthyle ; et U représente S, un groupe CH₂ ou une liaison covalente, comprenant la réaction d'un composé de formule 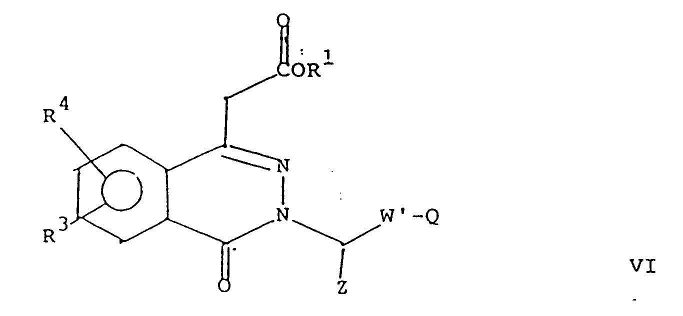 dans laquelle R¹, R³, R⁴ et Z répondent aux définitions précitées, W' représente S, un groupe CH₂ ou une liaison covalente, et Q représente un groupe -CN ou 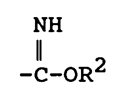 dans lequel R² représente un groupe alkyle en C₁ à C₄, ou d'un de ses sels d'addition d'acides, avec un composé de formule 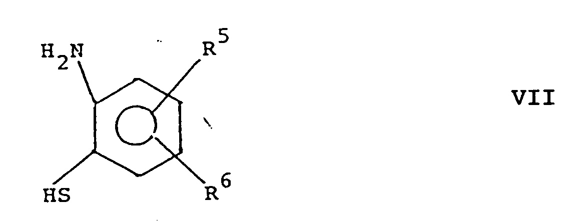 dans laquelle R⁵ et R⁶ répondent aux définitions précitées, ou avec un de ses sels d'addition d'acides, sous réserve que, lorsqu'aucun des composés de formule VI et de formule VII n'est sous forme de son sel d'addition d'acide lorsqu'il est ajouté à l'autre desdits composés, la réaction soit conduite en présence d'un acide fort ; et, si cela est désiré, l'hydrolyse d'un composé de formule VIII dans laquelle R¹ est autre que l'hydrogène en le composé correspondant de formule VIII dans laquelle R¹ représente l'hydrogène.

2. Procédé suivant la revendication 1, dans lequel le composé de formule VI dans laquelle Q représente un groupe -CN est préparé par réaction d'un composé de formule 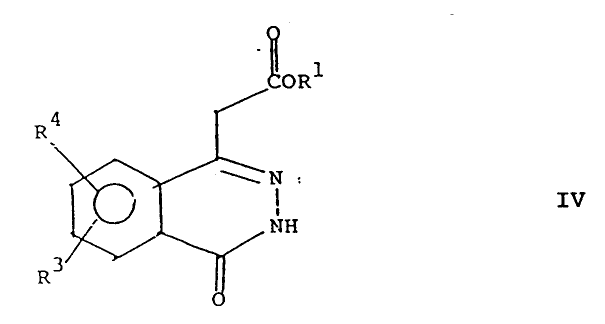 dans laquelle R¹, R³ et R⁴ répondent aux définitions suivant la revendication 1, avec un composé de formule 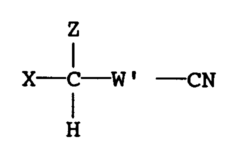 dans laquelle X représente un groupe chloro, bromo, -OSO₂-(alkyle en C₁ à C₄) ou -OSO₂-aryle dans lequel le groupe aryle est un groupe phényle, naphtyle, phényle substitué ou naphtyle substitué, les substituants sur les groupes phényle substitué et naphtyle substitué étant des groupes alkyle en C₁ à C₄, des halogènes ou des groupes nitro, en présence d'une base convenable.

3. Procédé suivant la revendication 1, dans lequel un composé de formule VI, dans laquelle W' représente une liaison covalente et Z représente l'hydrogène, est préparé par réaction d'un composé de formule 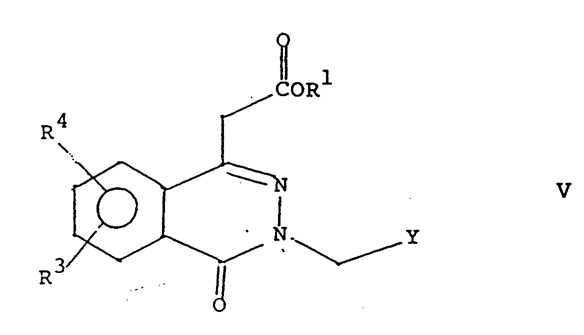 dans laquelle Y représente un groupe chloro ou bromo et R¹, R³ et R⁴ répondent aux définitions mentionnées pour la formule V dans la revendication 1, avec un cyanure de métal alcalin ou un cyanure de métal alcalino-terreux.

4. Procédé suivant la revendication 1, dans lequel un composé de formule VI est fondu conjointement avec un sel d'addition d'acide préformé d'un composé de formule VII à une température comprise dans l'intervalle d'environ 110 à environ 180°C.

5. Procédé de préparation d'un composé de formule 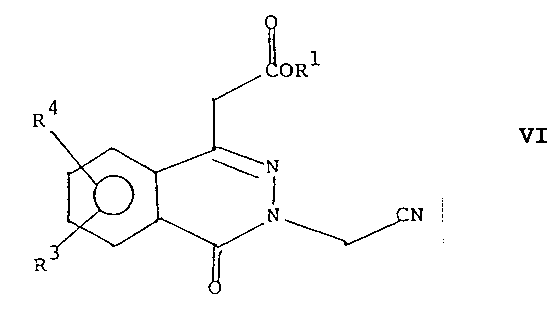 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent des groupes alcanedioxy en C₁ à C₄, comprenant la réaction d'un composé de formule 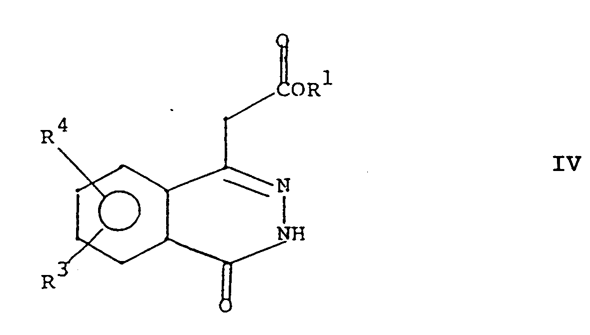 dans laquelle R¹, R³ et R⁴ répondent aux définitions précitées, avec un composé de formule XCH₂CN dans laquelle X représente un groupe chloro, bromo, -OSO₂-(alkyle en C₁ à C₄) ou -OSO₂-aryle dans lequel le groupe aryle représente un groupe phényle, naphtyle, phényle substitué ou naphtyle substitué, les substituants sur le groupe phényle substitué et le groupe naphtyle substitué étant des groupes alkyle en C₁ à C₄, des halogènes ou des groupes nitro ; Z représente l'hydrogène ou un groupe méthyle et W' représente un groupe CH₂ ou une liaison covalente, en présence d'une base convenable.

6. Procédé de préparation d'un composé de formule 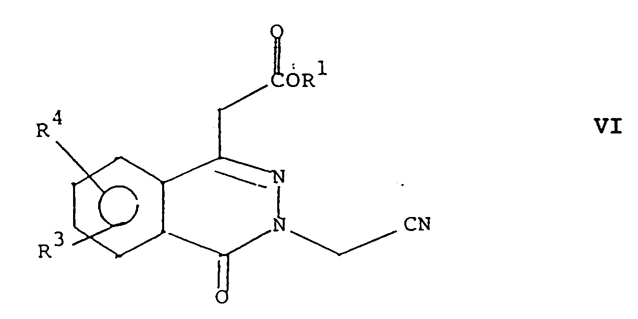 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄, comprenant la réaction d'un composé de formule 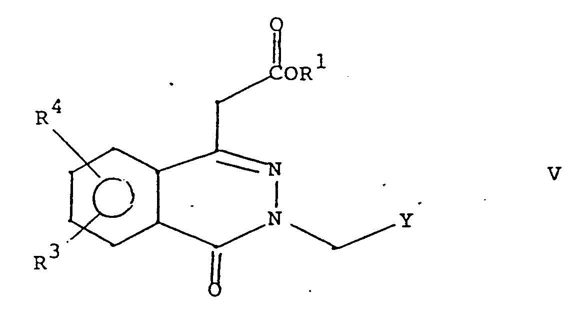 dans laquelle Y représente un groupe chloro ou bromo et R¹, R³ et R⁴ répondent aux définitions précitées, avec un cyanure de métal alcalin ou un cyanure de métal alcalino-terreux.

7. Procédé de préparation d'un composé de formule 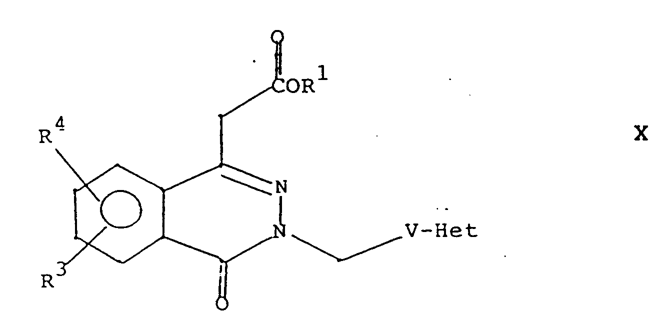 dans laquelle R¹ représente l'hydrogène ou un groupe alkyle en C₁ à C₄ ; R³ et R⁴ sont identiques ou différents et représentent l'hydrogène, des groupes fluoro, chloro, bromo, trifluorométhyle, alkyle en C₁ à C₄, alkoxy en C₁ à C₄, alkylthio en C₁ à C₄, alkylsulfinyle en C₁ à C₄, alkylsulfonyle en C₁ à C₄ ou nitro, ou bien R³ et R⁴, pris conjointement, représentent un groupe alcanedioxy en C₁ à C₄ ; V représente l'oxygène, le soufre ou un groupe NH ; et Het représente un noyau pentagonal hétérocyclique possédant un atome d'azote, d'oxygène ou de soufre, deux atomes d'azote dont l'un peut être remplacé par l'oxygène ou le soufre, ou trois atomes d'azote dont l'un peut être remplacé par l'oxygène ou le soufre, ledit noyau étant substitué avec un ou deux groupes fluoro, chloro, alkyle en C₁ à C₄ ou phényle, ou condensé avec un groupe benzo, ou substitué avec un groupe pyridyle, furyle ou thiényle, ledit groupe phényle ou benzo étant facultativement substitué avec un groupe iodo ou trifluorométhylthio, ou un ou deux groupes fluoro, chloro, bromo, alkyle en C₁ à C₄, alkoxy en C₁ à C₄, alkylthio en C₁ à C₄, alkylsulfinyle en C₁ à C₄, alkylsulfonyle en C₁ à C₄ ou trifluorométhyle, et ledit groupe pyridyle, furyle ou thiényle étant facultativement substitué en position 3 avec un groupe fluoro, chloro, bromo, alkyle en C₁ à C₄ ou alkoxy en C₁ à C₄ ; un noyau hexagonal hétérocyclique ayant 1 à 3 atomes d'azote, ou un ou deux atomes d'azote et un atome d'oxygène ou de soufre, et ledit noyau étant substitué avec un ou deux groupes alkyle en C₁ à C₄ ou phényle, ou condensé avec un groupe benzo, ou substitué avec un groupe pyridyle, furyle ou thiényle, ledit groupe phényle ou benzo étant facultativement substitué avec un groupe iodo ou trifluorométhylthio, ou un ou deux groupes fluoro, chloro, bromo, alkyle en C₁ à C₄, alkoxy en C₁ à C₄, alkylthio en C₁ à C₄, alkylsulfinyle en C₁ à C₄, alkylsulfonyle en C₁ à C₄ ou trifluorométhyle, et ledit groupe pyridyle, furyle ou thiényle étant facultativement substitué en position 3 avec un groupe fluoro, chloro, alkyle en C₁ à C₄ ou alkoxy en C₁ à C₄ ; un groupe oxazole ou thiazole condensé avec un groupe aromatique hexagonal contenant un ou deux atomes d'azote ou avec le thiophène ou le furanne, chacun faculattivement substitué avec un groupe fluoro, chloro, bromo, trifluorométhyle, méthylthio ou méthylsulfinyle ; imidazolopyridine ; naphtothiazole ; ou naphtoxazole ; comprenant la réaction d'un composé de formule 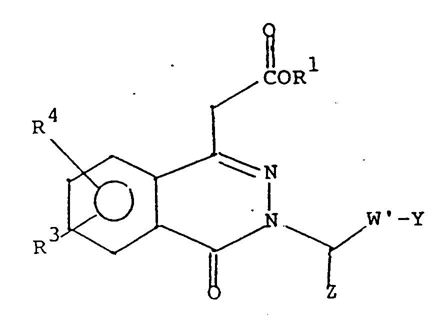 dans laquelle R¹, R³ et R⁴ répondent aux définitions précitées, W' représente une liaison covalente, Z représente H et Y représente un groupe chloro ou bromo, avec un composé de formule HV-Het dans laquelle V et Het répondent aux définitions précitées.
